(19) Europäisches Patentamt / European Patent Office / Office européen des brevets

(11) EP 3 653 662 B1

(12) EUROPÄISCHE PATENTSCHRIFT

(45) Veröffentlichungstag und Bekanntmachung des Hinweises auf die Patenterteilung:
**07.06.2023 Patentblatt 2023/23**

(21) Anmeldenummer: **19208538.9**

(22) Anmeldetag: **12.11.2019**

(51) Internationale Patentklassifikation (IPC):
***C08G 77/20*** (2006.01) ***B33Y 70/00*** (2020.01) ***B33Y 80/00*** (2015.01) ***C12N 5/00*** (2006.01) ***C12M 1/12*** (2006.01) ***G03F 7/075*** (2006.01) ***G03F 7/00*** (2006.01) ***B29C 64/124*** (2017.01) ***B29C 64/273*** (2017.01) ***A61L 27/18*** (2006.01) ***A61L 27/56*** (2006.01) ***C12M 1/26*** (2006.01) ***G03F 7/004*** (2006.01) ***G03F 7/027*** (2006.01) ***G03F 7/20*** (2006.01)

(52) Gemeinsame Patentklassifikation (CPC):
(C-Sets verfügbar)
**C08F 230/085; A61L 27/18; A61L 27/56; B33Y 70/00; B33Y 80/00; C12M 25/14; C12M 33/00; C12N 5/0068; G03F 7/0037; G03F 7/0047; G03F 7/027; G03F 7/0755; G03F 7/0757; G03F 7/2053;** C08F 220/10; (Forts.)

(54) **DREIDIMENSIONALE GERÜSTSTRUKTUREN MIT SELEKTIV FUNKTIONALISIERTEN POLYMERSYSTEMEN, DEREN VERWENDUNG UND VERFAHREN ZU DEREN HERSTELLUNG**

THREE-DIMENSIONAL FRAMEWORK STRUCTURES WITH SELECTIVELY FUNCTIONALISED POLYMER SYSTEMS, THEIR USE AND METHOD FOR THE PRODUCTION THEREOF

STRUCTURES D'OSSATURE TRIDIMENSIONNELLES COMPRENANT DES SYSTÈMES POLYMÈRES FONCTIONNALISÉS SÉLECTIVEMENT, LEUR UTILISATION ET LEUR PROCÉDÉ DE FABRICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.11.2018 DE 102018128576**

(43) Veröffentlichungstag der Anmeldung:
**20.05.2020 Patentblatt 2020/21**

(73) Patentinhaber: **Fraunhofer-Gesellschaft zur Förderung der angewandten Forschung e.V.**
**80686 München (DE)**

(72) Erfinder:
- **WOLTER, Herbert**
  **97082 Würzburg (DE)**
- **NIQUE, Somchith**
  **97082 Würzburg (DE)**
- **HASSELMANN, Sebastian**
  **97082 Würzburg (DE)**
- **KOPITTKE, Caroline**
  **1040 Wien (AT)**
- **HEINRICH, Doris**
  **97082 Würzburg (DE)**
- **SCHWAIGER, Johannes**
  **97082 Würzburg (DE)**

(74) Vertreter: **Strehl Schübel-Hopf & Partner**
**Maximilianstrasse 54**
**80538 München (DE)**

(56) Entgegenhaltungen:
**DE-A1- 10 349 766 DE-A1-102017 101 823**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 653 662 B1

(52) Gemeinsame Patentklassifikation (CPC): (Forts.)
C12N 2529/10; C12N 2533/30

C-Sets
**A61L 27/18, C08L 83/10**

## Beschreibung

### Gebiet der Erfindung

Die vorliegende Erfindung stellt eine dreidimensionale Gerüststruktur und ein Verfahren zu deren Herstellung bereit, wobei die Gerüststruktur ein Polymersystem umfasst, das durch Photostrukturierung eines Ausgangmaterials herstellbar ist, das Vorläufermoleküle oder anorganische Polymere davon enthält, die einen organisch polymeriserbaren Rest mit mindestens einer C=C-Doppelbindung, einen anorganisch polymersierbaren Rest auf Silanbasis sowie eine funktionelle Gruppe oder ein Derivat davon aufweist. Die erfindungsgemäße Gerüststruktur kann zur Wechselwirkung mit biologischen Zellen eingesetzt werden.

### Stand der Technik

Im Bereich der Zellkultur, des Tissue Engineering und Lab-on-a-Chip-Systemen benötigt man Gerüststrukturen (Scaffolds), um die natürliche Umgebung von biologischen Zellen zu imitieren und über die Umgebung das Zellverhalten wie Proliferation, Adhäsion oder Differenzierung gezielt zu steuern. Durch Variation der Oberflächenrauigkeit, der Strukturierung im Mikrometerbereich oder der chemischen Oberflächenfunktionalisierung ist es beispielsweise möglich, das Verhalten biologischer Zellen zu beeinflussen.

Es gibt im Stand der Technik zahlreiche Materialien, die als biologische Gerüststruktur eingesetzt werden können, z. B. Hydrogele, Gläser oder Fasern. Teilweise ist es auch möglich, diese mittels 3D-Druck-, Lithographie- oder MPP-Verfahren herzustellen. Das Polymerisationsverfahren mittels Mehr-Photonen-Polymerisation (MPP) oder Zwei-Photonen-Polymerisation (2PP) wird mittlerweile in vielen Bereichen eingesetzt.

Es gibt diverse Materialsysteme, die sich für die TPA-Strukturierung von Gerüststrukturen eignen, allerdings keine räumlich selektiv angepasste Oberflächenchemie aufweisen. Beispielsweise wurde ORMOCOMP® (Mehrfachacrylat-ORMOCER®) als mögliches TPAstrukturierbares Material für Zellanwendungen untersucht (Schlie S et al. (2007) Three-dimensional cell growth on structures fabricated from ORMOCER by two-photon polymerization technique. Journal of biomaterials applications 22:275-287).

DE102017101823A1 offenbart ein Komposit, das photostrukturiertes Matrixmaterial und darin enthaltene Nanopartikel umfasst, sowie die Verwendung des Komposits für medizinische oder biologische Zwecke.

Es gibt prinzipiell 2 Möglichkeiten der Funktionalisierung von Polymer-Gerüststrukturen, nämlich die Pre-Polymerisationsfunktionalisierung (das entspricht dem hier beanspruchten Ansatz) und die Post-Polymerisationsfunktionalisierung. Beispiele für Prepolymerisationsfunktionalisierungen UV-reaktiver organischer Harze finden sich u.a. bei (Meth)-Acrylaten z.B. mit NHR und OH Funktionalitäten. OH-haltige Acrylatmonomere finden sich in EP 2 870 209 B1. Sowohl bei OH bzw. NHR-präfunktionalisierten Polymeren sind bislang jedoch keine 3D-Gerüststrukturierungsverfahren beschrieben. Bei der Postfunktionalisierung sind verschiedenste Beschichtungen (z.B. Collagen) und Funktionalisierungsmethoden (z.B. Silanisierung, Fluorierung, Photoinduziertes Grafting, Plasmabehandlungen, Adsorption oder kovalente Anbindung von Proteinen und anderen bioaktiven Molekülen) sowie Bulk-Modifikation (z.B. direct mixing mit Biofunktionsmolekülen, Carriersysteme mit Mikro- und Nanopartikeln) möglich. Hierbei besteht allerdings das Problem, dass nur die gesamte Oberfläche der Gerüststruktur beschichtet werden kann. Es wird ein Material verwendet, das sich mit hoher Auflösung photostrukturieren lässt und in einem weiteren Prozessschritt überschichtet wird und so an der gesamten Oberfläche funktionalisiert ist.

Es sind Beispiele für selektive Funktionalisierungen von Polymer-Gerüststrukturen bekannt. In der Literatur werden Kombinationen verschiedener Materialsysteme innerhalb einer Probe beschrieben. Die Kombination von TPA-strukturiertem PDMS, SZ2080, ORMOCLEAR®, PEG-DA innerhalb einer Probe ist in (Rekstyte S (2014) Direct Laser Fabrication of Composite Material 3D Microstructured Scaffolds. JLMN 9:25-30) beschrieben. Die Kombination von PEG und ORMOCOMP® innerhalb einer Probe ist in (Klein F et al. (2011) Two-component polymer scaffolds for controlled three-dimensional cell culture. Advanced materials (Deerfield Beach, Fla.) 23:1341-1345) beschrieben, wobei gezeigt werden konnte, dass die Zellen hauptsächlich an ORMOCOMP® adhärieren und so die Zelladhäsion und Zellmorphologie gesteuert werden kann. Allerdings ist hier das Basismaterial nicht identisch, was zu stark unterschiedlichen Prozessschritten und damit zu komplizierteren Prozessverfahren führen kann.

Es wurden auch bereits mehrere Materialien innerhalb einer Probe kombiniert und diese Materialien in einem weiteren Prozessschritt mit unterschiedlichen, biologisch aktiven Gruppen an der Oberfläche funktionalisiert. Chemisch wurde das so abgestimmt, dass die unterschiedlichen Substanzen nur an einem Material binden können und die Proben so lokal unterschiedliche Oberflächenchemie aufweisen und deshalb die Adhäsion der Zellen örtlich selektiv beeinflusst werden kann (Richter B et al. (2017) Guiding Cell Attachment in 3D Microscaffolds Selectively Functionalized with Two Distinct Adhesion Proteins. Advanced materials (Deerfield Beach (Fla.) 29). Da es sich hierbei nicht um eine intrinsische Funktionalisierung handelt, liegt diese nur an der Oberfläche vor und muss aufwändig durch zusätzliche Prozessschritte realisiert werden.

## Durch die Erfindung zu lösende Aufgaben

Ziel im Tissue Engineering ist es, das Zellverhalten, z. B. Proliferation, Adhäsion, Differenzierung oder Migration, durch externe Stimuli gezielt zu steuern. Dabei spielt die Beschaffenheit des Zellsubstrats eine entscheidende Rolle. Um die natürliche Zellumgebung in vitro zu simulieren, sollten dreidimensionale Gerüststrukturen (Scaffolds) definierte Eigenschaften wie Oberflächenchemie, Elastizität, Rauheit, Topographie und Dimensionalität haben. Die chemische Funktionalisierung der Gerüstoberfläche nimmt dabei unter anderem Einfluss auf die Proliferations- und Adhäsionsfähigkeit der Zellen. Durch die Verwendung geeigneter chemischer Gruppen lässt sich außerdem die Anbindung von Molekülen aus dem Zellmedium steuern, die bei der Zelladhäsion vermitteln. Für komplexere biologische Systeme sind selektiv strukturierte Gerüststrukturen nötig, die durch lokal variierende Eigenschaften, z. B. des E-Moduls, der Oberflächenfunktionalisierung, Oberflächenpolarität, Fluoreszenz und Oberflächenrauheit, zu einer Beeinflussung des Zellverhaltens führen.

Es besteht also großer Bedarf an einer dreidimensionalen Gerüststruktur, die eine Zellumgebung in vitro simulieren kann, selektiv strukturiert sein kann und chemische Funktionalisierungen auf der Gerüstoberfläche aufweist, so dass das Zellverhalten in gewünschter Weise gesteuert werden kann.

## Zusammenfassende Darstellung der Erfindung

Die der Erfindung zugrunde liegende Aufgabe wurde durch die Bereitstellung der erfindungsgemäßen dreidimensionalen Gerüststruktur gelöst. Diese Struktur umfasst ein Polymersystem, das aus Vorläufermolekülen hergestellt werden kann, die drei Funktionen aufweisen, nämlich organische Polymerisierbarkeit durch Photostrukturierung, anorganische Polymerisierbarkeit durch Hydrolyse/Kondensation und Bioaktivität durch Funktionalisierung bzw. Derivatisierung.

Die vorliegende Erfindung betrifft eine 3D-Gerüststruktur, die Verwendung der 3D-Gerüststruktur sowie ein Verfahren zur Herstellung der 3D-Gerüststruktur gemäß den beigefügten Ansprüchen.

[1] Dreidimensionale Gerüststruktur, umfassend ein Polymersystem, das durch Photostrukturierung eines Ausgangmaterials herstellbar ist, das Vorläufermoleküle der Formel (I) oder anorganische Polymere davon enthält:

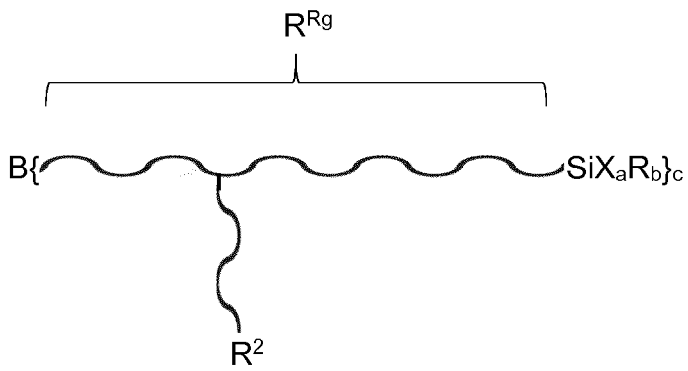

Formel (I)

worin die Reste und Indices folgende Bedeutung haben:

B ist ein geradkettiger oder verzweigter oder cyclischer organischer Rest mit mindestens einer C=C-Doppelbindung und 4 bis 100 Kohlenstoffatomen;
X ist Wasserstoff, Halogen, Hydroxy, Alkoxy, Acyloxy, Alkylcarbonyl, Alkoxycarbonyl oder $NR^4_2$;
R ist Alkyl, Alkenyl, Aryl, Alkylaryl oder Arylalkyl;
$R^2$ ist OH, COOH, $NR^4_2$, $NR^4_2H^+$ oder SH oder ein Derivat davon;
$R^4$ ist Wasserstoff, Alkyl, Aryl oder Alkylaryl;
$R^{Rg}$ ist das Rückgrat eines an das Si-Atom gebundenen geradkettigen oder verzweigten Kohlenwasserstoffes, wobei das Rückgrat durch Heteroatome oder heteroatomhaltige Gruppen unterbrochen sein kann;
a = 1, 2 oder 3;
b = 0, 1 oder 2;

$$a+b = 3;$$

c = 1, 2, 3 oder 4.

Die dreidimensionale Gerüststruktur nach Punkt [1] weist vorzugsweise Gerüststrukturteile aus einem Polymersystem und zwischen den Gerüststrukturteilen fluidgefüllte offene Räume mit einem Mindestmaß von 50 nm in jeder Raumrichtung auf, wobei das Polymersystem durch Behandlung eines Ausgangsmaterials mit elektromagnetischer Strahlung herstellbar ist, wobei das Ausgangsmaterial Vorläufermoleküle der Formel (I) oder anorganische Polymere davon enthält.

Der Ausdruck "Heteroatom" bedeutet beispielsweise -O-, -NH-, oder -S-. Der Ausdruck "heteroatomhaltige Gruppen" bedeutet beispielsweise solche, die mindestens zwei Heteroatome und höchstens ein Kohlenstoffatom enthalten, wie -O-CO-, -S-CO-, -NH-CO- und -CO-O. Das Kohlenwasserstoffrückgrat kann also beispielsweise durch ein oder mehrere Mitglieder der Gruppe unterbrochen sein, die aus -O-, -NH-, -S-, -O-CO-, -S-CO-, - NH-CO- und -CO-O- besteht.

[2] Dreidimensionale Gerüststruktur nach Punkt [1], wobei das Ausgangsmaterial Vorläufermoleküle der Formel (II) oder anorganische Polymere davon enthält:

$$B\{A\text{-}(Z)_d\text{-}R^1\text{-}R^3\text{-}SiX_aR_b\}_c \qquad \text{Formel (II)}$$
$$\quad |$$
$$(O\text{-}CO\text{-}R\text{-})_eR^2$$

worin die Reste und Indices folgende Bedeutung haben:

B, X, R, $R^2$, $R^4$, a, b, a+b und c haben die gleiche Bedeutung wie in Formel (I);
$R^1$ und $R^3$ sind unabhängig voneinander Alkylen, Arylen oder Alkylenarylen mit jeweils 1 bis 20 Kohlenstoffatomen, wobei diese Reste durch O, S, NR oder NH unterbrochen sein können,
A ist O, S oder NH für d = 1 und Z = CO; oder A ist O, S, NH oder COO für d = 1;
Z ist $CHR^4$; oder A ist O, S, NH oder COO für d = 0; und
e = 0 oder 1.

In Punkt [2] ist es bevorzugt, dass die Reste und Indices der allgemeinen Formel (II) vorzugsweise die folgende Bedeutung haben: X ist ($C_1$-$C_4$)-Alkoxy oder Halogen, R ist ($C_1$-$C_4$)-Alkyl und $R^3$ ist ($C_1$-$C_4$)-Alkylen.

Besonders bevorzugt ist eine 3D-Gerüststruktur nach Punkt [2], worin gilt: c ist 1, d ist 0, e ist 0, $R^2$ ist OH, X ist ($C_1$-$C_4$)-Alkoxy oder Halogen, R ist ($C_1$-$C_4$)-Alkyl, $R^3$ ist ($C_1$-$C_4$)-Alkylen und der organische Rest BA ist von Acrylat oder Methacrylat abgeleitet.

[3] Dreidimensionale Gerüststruktur nach Punkt [1] oder [2],

wobei das Polymersystem auf mindestens eine erste Art von Vorläufermolekülen der Formel (I) bzw. (II) oder anorganischen Polymeren davon sowie auf mindestens eine zweite Art von Vorläufermolekülen oder anorganischen Polymeren davon zurückgeht, wobei sich die zweite Art von der ersten Art mindestens oder ausschließlich darin unterscheidet, dass $R^2$ Wasserstoff ist; oder
wobei das Polymersystem auf mindestens zwei Arten von Vorläufermolekülen oder anorganischen Polymeren davon zurückgeht, die sich mindestens oder ausschließlich in der Gruppe $R^2$ unterscheiden.

Es besteht die Möglichkeit, dass sich die zwei Arten mindestens oder ausschließlich in der Gruppe $R^2$ unterscheiden. Der Unterschied kann in der Art der Funktionalisierung, also ob $R^2$ unter OH, COOH, $NR^4_2$, $NR^4_2H^+$ oder SH ausgewählt ist, und/oder in der Art der Derivatisierung bestehen, also welche Art eines Biofunktionsmoleküls an $R^2$ gebunden worden ist.

[4] Dreidimensionale Gerüststruktur nach Punkt [3], wobei der Unterschied darin besteht, dass in der einen Art des Vorläufermoleküls $R^2$ OH, COOH, $NR^4_2$, $NR^4_2H^+$ oder SH ist und in der anderen Art des Vorläufermoleküls $R^2$ ein Derivat von $R^2$ der ersten Art ist.

[5] Dreidimensionale Gerüststruktur nach einem der vorstehenden Punkte, wobei das Derivat von $R^2$ auf ein Biofunktionsmolekül oder auf einen Abstandshalter mit angebundenem Biofunktionsmolekül zurückgeht.

[6] Dreidimensionale Gerüststruktur nach einem der vorstehenden Punkte, wobei das Polymersystem Nanopartikel enthält.

[7] Dreidimensionale Gerüststruktur, die zwei Gerüststrukturteile enthält, die in einem der vorstehenden Punkte beschriebene Polymersysteme enthalten, wobei sich die Polymersysteme in mindestens einem Merkmal unterscheiden, das aus der Gruppe ausgewählt ist, bestehend aus der Art des Vorläufermoleküls der Formel (I) bzw. (II) oder eines anorganischen Polymers davon; dem Anteil des Vorläufermoleküls der Formel (I) bzw. (II) oder eines anorganischen Polymers davon an den gesamten photostrukturierbaren Verbindungen; dem Anteil eines Vorläufermoleküls, das mit Ausnahme von $R^2$=H dem eingesetzten Vorläufermolekül der Formel (I) bzw. (II) entspricht; dem Gehalt an Nanopartikeln; dem Anteil des Vorläufermoleküls der Formel (I) bzw. (II), worin $R^2$ ein Derivat ist; und der Art des Derivats an $R^2$.

[8] Dreidimensionale Gerüststruktur nach einem der vorstehenden Punkte, worin die Polymersysteme aus einer einzigen Art eines Vorläufermoleküls herstellbar sind, mit der Ausnahme, dass sich die Vorläufermoleküle in dem Vorhandensein und der Art der Derivatisierung an $R^2$ unterscheiden können.

[9] Dreidimensionale Gerüststruktur nach einem der Punkte [1] bis [7], die Gerüststrukturteile aus einem Polymersystem, das im Ausgangsmaterial erste Vorläufermoleküle der Formel (I) bzw. (II) oder anorganische Polymere davon enthält, und Gerüststrukturteile aus einem Polymersystem umfasst, das im Ausgangsmaterial von den ersten verschiedene zweite Vorläufermoleküle der Formel (I) bzw. (II) oder anorganische Polymere davon enthält.

[10] Dreidimensionale Gerüststruktur nach einem der vorstehenden Punkte, die mindestens eine erste Struktureinheit einer aus dem Bereich von 10 μm bis 100 mm ausgewählten ersten Dicke und von der ersten Struktureinheit abzweigende zweite Struktureinheiten einer jeweils aus dem Bereich von 100 nm bis 1000 μm ausgewählten zweiten Dicke enthält, wobei an den Abzweigungen die zweite Dicke höchstens die Hälfte der ersten Dicke ist.

[11] Verwendung der dreidimensionalen Gerüststruktur nach einem der vorstehenden Punkte für die Wechselwirkung mit biologischen Zellen in vitro.

[12] Verfahren zum Herstellen einer dreidimensionalen Gerüststruktur nach einem der Punkte [1] bis [11], welches die folgenden Schritte umfasst:

(a) Bereitstellen eines in Punkt [1] oder [2] beschriebenen photostrukturierbaren Ausgangsmaterials,
(b) Photostrukturierung des in Schritt (a) bereitgestellten Ausgangsmaterials unter Bildung einer 3D-Gerüststruktur und
(c) Entfernen des nicht umgesetzten Ausgangsmaterials.

[13] Verfahren nach Punkt [12], welches einen ersten Durchgang der Schritte (a) bis (c) mit einer ersten Position der Photostrukturierung und einen zweiten Durchgang der Schritte (a) bis (c) mit einer von der ersten Position verschiedenen zweiten Position der Photostrukturierung unter Bildung von zwei Teilstrukturen der dreidimensionalen Gerüststruktur umfasst.

Die Position der zweiten Photostrukturierung wird dabei vorzugsweise so gewählt, dass durch die Photostrukturierung die zwei Teilstrukturen kovalent verbunden werden. Die Positionen der beiden Photostrukturierungen sind also vorzugsweise aneinander angrenzend. Dabei können die beiden Teilstrukturen der gleichen oder unterschiedlichen Hierarchieebenen angehören. Die Art der funktionellen Gruppe $R^2$ ist ausgewählt unter OH, COOH, $NR^4_2$, $NR^4_2H^+$ oder SH, wobei $R^4$ Wasserstoff, Alkyl, Aryl oder Alkylaryl ist. Das Derivat ist erhältlich durch Reaktion der Gruppe $R^2$ mit einem in der vorliegenden Beschreibung genannten Biofunktionsmolekül oder einem Abstandshalter-modifizierten Abkömmling davon.

[14] Verfahren nach Punkt [13], wobei sich die Ausgangsmaterialien des ersten Durchgangs von denen des zweiten Durchgangs in mindestens einem Merkmal unterscheiden, das aus der Gruppe ausgewählt ist, bestehend

aus der Art des Vorläufermoleküls der Formel (I) bzw. (II) oder eines anorganischen Polymers davon;
dem Anteil des Vorläufermoleküls der Formel (I) bzw. (II) oder eines anorganischen Polymers davon an den gesamten photostrukturierbaren Verbindungen;
dem Anteil eines Vorläufermoleküls, das mit Ausnahme von $R^2$=H dem eingesetzten Vorläufermolekül der Formel (I) bzw. (II) entspricht;
dem Gehalt an Nanopartikeln; dem Anteil des Vorläufermoleküls der Formel (I) bzw. (II), worin $R^2$ ein Derivat ist; und der Art des Derivats an $R^2$ besteht.

Hier umfasst der Unterschied im "Anteil" auch die Möglichkeit, dass der betreffende Bestandteil im Ausgangsmaterial eines Durchgangs enthalten und im anderen überhaupt nicht enthalten ist.

In einer bevorzugten Ausführungsform unterscheiden sich die Vorläufermoleküle oder die anorganischen Polymere davon in den beiden Durchgängen in der Art der funktionellen Gruppe $R^2$ oder in dem Derivat der Gruppe $R^2$ oder in beiden.

[15] Verfahren nach Punkt [13] oder [14], wobei nur eine einzige Art des Vorläufermoleküls der Formel (I) bzw. (II) oder eines anorganischen Polymers davon eingesetzt wird, mit der Ausnahme, dass sich die Vorläufermoleküle in dem Vorhandensein und der Art der Derivatisierung an $R^2$ unterscheiden können, und wobei diese Art in beiden Durchgängen gleich oder verschieden ist.

Indem in beiden Durchgängen nur eine einzige Art des Vorläufermoleküls der Formel (I) bzw. (II) eingesetzt wird, kann das Verfahren vereinfacht werden, während gleichzeitig durch die unterschiedlichen Konzentrationen und Derivatisierungen der Vorläufermoleküle, durch die Reihenfolge der Verfahrensschritte, durch die Form und die Ausmaße der Teilstrukturen und Anwesenheit von Nanomolekülen große Variationsmöglichkeiten bezüglich der Strukturen und Eigenschaften bestehen.

## Vorteile der Erfindung

Die Funktionalisierung und/oder Derivatisierung der Vorläufermoleküle führt dazu, dass die gewünschten Gruppen gleichmäßig in die erfindungsgemäße 3D-Gerüststruktur eingebaut werden können und somit durchgehend, nämlich an der Oberfläche und im Inneren, in einem Strukturteil vorhanden sein können.

Durch die intrinsische Funktionalisierung lassen sich hochaufgelöste makroskopische Gerüststrukturen in Verbindung mit unterschiedlichen Funktionalisierungsvarianten bei gleicher chemischer Grundstruktur herstellen.

Die entwickelten organisch funktionalisierten, anorganischen Polymersysteme sind biokompatibel, intrinsisch funktionalisiert und mit allen gängigen Methoden photostrukturierbar. Durch die Kombination dieser Verfahren ist es möglich beliebig 3D-geformte Gerüststrukturen im Zentimeterbereich mit einer Strukturierungsauflösung bis in den Submikrometer-Bereich herzustellen. Durch die intrinsische Funktionalisierung lassen sich durch mehrere aufeinanderfolgende Strukturierungsschritte Gerüststrukturen mit lokal variierter Funktionalisierung herstellen.

Des Weiteren ist es möglich an die funktionellen Gruppen in einem weiteren Prozessschritt bestimmte bioaktive Stoffe wie Proteine oder ähnliches kovalent zu binden (z. B. Biotin-Avidin-Kopplung). Durch die selektive Strukturierung können diese Stoffe räumlich variiert vorliegen.

Durch Zugabe von Nanopartikeln ist es möglich, zusätzlich eine intrinsische Oberflächenrauheit im Nanometerbereich ohne weiteren Prozessschritt zu realisieren. Diese kann durch den Durchmesser der Nanopartikel beliebig variiert und an jeden Zelltyp bzw. das gewünschte Zellverhalten angepasst werden.

Durch die Affinität verschiedener Zellen für bestimmte Umgebungseigenschaften erlauben selektive Gerüststrukturen deshalb die gezielte Steuerung des Zellmigrations- und Ansiedelungsverhaltens in verschiedenen Bereichen der Gerüststruktur.

Die Verbindung von Biokompatibilität, Photostrukturierbarkeit und insbesondere die definiert in großer Variabilität einstellbare intrinsische Funktionalisierung ermöglicht die Herstellung hochaufgelöster Zellgerüststrukturen mit lokal einstellbaren chemischen und physikalischen Eigenschaften. Dadurch kann beispielsweise gezielt auf die Stammzelldifferenzierung Einfluss genommen werden.

Die erfindungsgemäßen 3D-Gerüststruktur kann beispielsweise für die Unterscheidung bzw. Sortierung verschiedener, zur Entwicklung von Body-on-a-chip-Systemen bis hin zur Herstellung künstlicher Organe mit mehreren Zelltypen genutzt werden.

Durch einen hierarchischen Aufbau der 3D-Gerüststruktur kann die Genauigkeit der Strukturteile erhöht werden. Gleichzeitig kann eine höhere Konzentration von Nanopartikeln unter Beibehaltung der Genauigkeit der Struktur eingesetzt werden.

Durch den Aufbau als hierarchische Struktur lassen sich Systeme bereitstellen, die einerseits sehr stabil sind und andererseits eine hohe innere Oberfläche aufweisen. Zudem lassen sich durch die Kombination von verschiedenen Strukturierungsverfahren Proben deutlich zeit- und kosten effizienter herstellen.

## Beschreibung der Figuren

Figur 1 zeigt die Herstellung dünner glatter Hybridpolymerschichten. Ein Präpolymertropfen wird zwischen zwei Präzisionsmikroskopdeckgläsern zu einer dünnen Schicht gepresst (links) und danach mit UV-Einstrahlung ausgehärtet (rechts). Nach dem Vernetzungsschritt kann das nicht-silanisierte Deckglas abgezogen werden, wobei eine dünne glatte Polymerschicht auf dem silanisierten Deckglas zurückbleibt.

Figur 2 zeigt die Herstellung von Mikrostrukturen mittels Zwei-Photonen-Polymerisation. A) Durch die Fokussierung eines gepulsten Laserstrahls in ein Hybridpolymer, das sich zwischen zwei Deckgläsern befindet, kommt es im Bereich des Objektivfokus zu einer Vernetzungsreaktion, und durch dreidimensionale Bewegung können beliebige Mikrostrukturen erzeugt werden (hier: Säulen, die in einem rechteckigen Muster angeordnet sind). B) Nach der Strukturierung werden die unvernetzten Anteile des Harzes mit einem Lösungsmittel wegentwickelt. C) Um den Vernetzungsgrad des Polymers zu erhöhen, wird die Struktur einer UV Nachbelichtung unterzogen. D) Auf den Strukturen werden Zellen ausgesät und deren Migrationsverhalten untersucht. Werden unterschiedlich funktionalisierte Polymere innerhalb einer Probe vereint, wird nach dem Entwicklungsschritt (B) die Struktur in einem weiteren Harz eingebettet und der Strukturierungsvorgang (E), die Entwicklung im Lösungsmittelbad (F) sowie die UV-Nachbelichtung wiederholt, bevor Zellen darauf ausgesät werden können (H).

Figur 3 zeigt die Auswertung der Zelltrajektorien mit lokaler MSD-Analyse. Zur Charakterisierung des Zellmigrationsverhaltens werden die gemessenen Zelltrajektorien $R(t_i)$ (links) einer lokalen Mean-Squared-Displacement-Analyse (MSD-Analyse) unterzogen.

Figur 4 zeigt den Einfluss der Funktionalisierung der Polymersysteme in 2D im Vergleich zu Glas. Die Verteilung des MSD-Exponenten $\alpha$ gibt Auskunft über die auftretenden Bewegungsmodi der beobachteten Zellen. Für $\alpha \lesssim 1{,}75$ befindet sich die Zelle im ungerichteten Bewegungsmodus, für $\alpha \gtrsim 1{,}75$ führt sie eine gerichtete Bewegung aus. Im Vergleich zu Glas zeigt sich auf den untersuchten Polymersystemen eine deutliche Verschiebung zum subdiffusiven Bereich ($\alpha$<1). Die Amin/OH-Funktionalisierung ($R^2$ = - $N(C_2H_5)_2$/-OH) bewirkt im Vergleich zur Hydroxyfunktionalisierung ($R^2$ = -OH) eine deutliche Zunahme des ungerichteten Bewegungsmodus.

Figur 5 zeigt den Einfluss der Funktionalisierung der Polymersysteme in quasi-3D. TPA-strukturierte Säulenfelder aus Hydroxy- bzw. Amin/OH-funktionalisiertem Polymersystem regen im Vergleich zu zweidimensionalen Schichten (Figur 4) den gerichteten Bewegungsmodus mit $\alpha$ > 1,75 an. Wieder ergibt sich für das Amin/OH-funktionalisierte Polymersystem ein geringerer Anteil gerichteter Bewegungen.

Figur 6 zeigt ein TPA-strukturiertes Säulenfeld aus OH- bzw. Amin/OH-funktionalisiertem Polymersystem. Links: Fluoreszenzmikroskopieaufnahme des Säulenfeldes. Das Amin/OHfunktionalisiertem Polymersystem fluoresziert stärker als das OH-funktionalisierte. Das Gradientenmuster auf der rechten Seite des Säulenfeldes ermöglicht den Zellen an jeder Stelle die Entscheidung zwischen beiden Funktionalisierungen. Rechts: Die markierte Zelle von D. *discoideum* (Pfeil) zeigt eine verstärkte Affinität zu den Amin-funktionalisierten Säulen.

Figur 7 zeigt eine TPA-strukturierte 3D-Gerüststruktur aus funktionalisiertem Polymersystem ($R^2$ = -OH). Links: REM-Aufnahme der Gerüststruktur. Rechts: Konfokale Fluoreszenzmikroskopieaufnahme von fluoreszenzmarkierten Zellen von D. *discoideum,* welche die Gerüststruktur besiedeln.

Figur 8A zeigt eine Fluoreszenzmikroskopaufnahme von D. *discoideum* Zellen, die innerhalb eines Feldes aus 2PP-strukturierten Säulen (Höhe: 10 $\mu$m, Durchmesser: 4 $\mu$m) migrieren. Die Säulen sind in einem Schachbrettmuster angeordnet und bestehen alternierend aus intrinsisch Hydroxy- und intrinsisch Amin-funktionalisiertem ORMOCER$^R$. Figuren 8B und 8C sind Histogramme der Berührungsereignisse von Zellen mit den Säulenoberflächen in Abhängigkeit der dabei vorhandenen Kontaktfläche für (B) Hydroxy- und (C) Amin-funktionalisierte Säulen.

Figur 9 zeigt die Bestimmung der Oberflächenrauheit von 2PP-strukturierten Säulen mittels Rasterkraftmikroskopie. A) Mikroskopaufnahme von Amin-funktionalisierten Säulen, die umgelegt wurden, um die seitliche Oberflächenrauheit zu vermessen. B) Rasterkraftmikroskopaufnahme einer Säule. C) Nach Abzug der Oberflächenkrümmung ergibt sich deren Oberflächenrauheit. D) Rasterelektronenmikroskopaufnahmen von je einer Säule aus Hydroxy-, Carboxy- und Amin-funktionalisiertem Hybridpolymer.

Figur 10 zeigt Röntgenphotoelektronenspektroskopie-Aufnahmen von 2PP strukturierten Oberflächen. Dargestellt ist jeweils der Kohlenstoff (1s) und Stickstoff (1s) Bereich für das mit Hydroxy (A, D), Carboxy (B, E), und Amin (C, F) funktionalisierte Polymer.

## Ausführungsformen der Erfindung

### Dreidimensionale Gerüststruktur

Im Rahmen der vorliegenden Erfindung bedeutet die erfindungsgemäße "dreidimensionale Gerüststruktur",

die im Folgenden auch einfach als "3D-Gerüststruktur" bezeichnet werden kann, eine dreidimensionale Struktur, die vorzugsweise Gerüststrukturteile aus einem Polymersystem und zwischen den Gerüststrukturteilen fluidgefüllte offene Räume aufweist. Die Gerüststrukturteile sind fest. Das Fluid ist ein bei Raumtemperatur gasförmiger oder flüssiger Stoff. Diese fluidgefüllten Räume sind Hohlräume oder Zwischenräume (insbesondere bei quasi-dreidimensionalen Strukturen). Die fluidgefüllten Räume sind nicht geschlossen, sondern zur Oberfläche der Gerüststruktur hin offen und stellen somit offene Poren in der Gerüststruktur dar. Das heißt, das Fluid und andere Stoffe können sich zwischen den fluidgefüllten Räumen und der Umgebung der Gerüststruktur bewegen. Dadurch sind die fluidgefüllten Räume und damit die Substituenten $R^2$ des Polymersystems für Stoffe aus der Umgebung der Gerüststruktur zugänglich. Vorzugsweise ist die Gerüststruktur so ausgestaltet, dass die fluidgefüllten Räume für die Stoffe zugänglich sind, die mit dem Substituenten $R^2$ des Polymersystems wechselwirken sollen. Wenn $R^2$ beispielsweise ein Enzym ist, ist die Gerüststruktur derart ausgestaltet, dass die fluidgefüllten Räume für ein Substrat des Enzyms zugänglich sind. Das Fluid ist vorzugsweise ein flüssiges Medium, beispielsweise ein Zellkulturmedium. Das strukturierte Polymer ist vorzugsweise vernetzt und stellt die Gerüststrukturteile der 3D-Gerüststruktur dar. Die Mindestdicke der Gerüststrukturteile wird bestimmt durch die Auflösung der Photostrukturierung. Diese Auflösung kann bis zu 10 nm sein, so dass das Mindestmaß der Gerüststrukturteile in jeder Raumrichtung mindestens 10 nm ist. Beispielsweise ist das Mindestmaß 10 nm bis 10 mm, vorzugsweise 1 $\mu$m bis 1000 $\mu$m. Die fluidgefüllten Räume zwischen den Gerüststrukturteilen haben ein Mindestmaß von 50 nm, vorzugsweise 100 nm, stärker bevorzugt 1000 $\mu$m in jeder Raumrichtung, wobei ein Bereich von 1 $\mu$m bis 1000 $\mu$m bevorzugt und ein Bereich von 1 $\mu$m bis 50 $\mu$m stärker bevorzugt sind. Bevorzugt ist eine Kombination von Gerüststrukturteilen mit einem Mindestmaß von 1 bis 1000 $\mu$m in jeder Raumrichtung und fluidgefüllte Räume mit einem Mindestmaß von 1 bis 50 $\mu$m in jeder Raumrichtung.

Die erfindungsgemäße Gerüststruktur ist vorzugsweise so ausgestaltet, dass sie bei Raumtemperatur nach Befüllung mit Wasser als Fluid zu mindestens 50 Volumenprozent aus Wasser bestehen kann.

Im Rahmen der vorliegenden Erfindung bedeutet "dreidimensional" die räumliche Ausdehnung einer einstückigen Struktur in alle drei Raumkoordinaten. Die Ausdehnung kann im Wesentlichen gleichmäßig in diesen drei Richtungen vorliegen, sodass zum Beispiel eine zylindrische Form, eine säulen-, quader- oder würfelförmige Matrixstruktur vorliegt. Es ist aber zum Beispiel auch möglich, dass die Ausdehnung in zwei Richtungen in größerem Ausmaß vorliegt, in die dritte Richtung jedoch nur in geringem Maße, so dass die dreidimensionale Struktur flächig wirkt, zum Beispiel eine Membran oder Schicht darstellt. Für die einzelnen Struktureinheiten ist eine solche Flächenform bevorzugt, das heißt, sie weisen eine Länge x, eine Breite y und eine Dicke z auf, wobei x und y jeweils mindestens doppelt, vorzugsweise mindestens dreimal und stärker bevorzugt mindestens fünfmal so groß wie z sind.

Neben den einstückigen räumlichen Strukturen umfasst der Ausdruck "dreidimensionale Strukturen" aber auch Strukturteile, die einander benachbart, jedoch ohne direkten Kontakt zueinander angeordnet sind. Die dadurch gebildete Gesamtstruktur wird auch als "quasidreidimensionale" Gerüststruktur bezeichnet. Wenn nichts anderes angegeben ist, umfasst der Ausdruck "dreidimensional" auch den Ausdruck "quasi-dreidimensional".

Im Rahmen der vorliegenden Erfindung bedeutet der Begriff "biokompatibel", dass die Gerüststruktur und die anderen Komponenten, beispielsweise ggf. enthaltene Nanopartikel, sowohl hinsichtlich ihrer Materialzusammensetzung als auch hinsichtlich ihrer Struktur für die Zellen oder das Gewebe keine toxische, apoptotische, unerwünschte immunologische oder sonstige unerwünschte Reaktion hervorruft und zelluläre und molekulare Prozesse auch nach einer möglichen Internalisierung von Nanopartikeln oder einem Abbau von Nanopartikeln und/oder Gerüststruktur nicht oder kaum stört.

Die 3D-Gerüststruktur kann eine große Bandbreite von Geweben simulieren, nämlich von Weichgeweben (0,4 bis 350 MPa), wie Gehirn und Bindegewebe bis zu Hartgeweben (10 bis 1500 MPa), wie Knorpel und Knochen.

Die Struktur kann zum Wechselwirken, z. B. Anhaften, von Zellen eingesetzt werden. Eine Gerüststruktur im Rahmen der vorliegenden Erfindung ist insbesondere in bevorzugter Ausführung eine Struktur, die nicht allein ein oberflächliches Anhaften oder Adhärieren von Zellen gestattet, sondern darüber hinaus insbesondere auch ein Hineinwachsen oder Integrieren von Zellen in die Gerüststruktur selbst.

Photostrukturierbarkeit

Das für die Herstellung der erfindungsgemäßen 3D-Gerüststruktur verwendete Ausgangsmaterial ist photostrukturierbar. Der hier verwendete Ausdruck "photostrukturierbar" bedeutet, dass das betreffende Material durch Behandlung mit elektromagnetischer Strahlung polymerisiert werden kann. Die elektromagnetische Strahlung kann beispielsweise durch ein fokussiertes Licht oder durch einen Laserstrahl gebildet sein.

Beispiele für Photostrukturierungsverfahren sind Rapid-prototyping, 3D-Druck, Lithographie und MPP.

Vorläufermoleküle und anorganische Polymere davon

Die erfindungsgemäßen 3D-Gerüststrukturen basieren auf organisch funktionalisierten anorganischen Polymerstrukturen. Sie beruhen im Wesentlichen auf ≡Si-O-Si≡ und sind ggf. mit Heteroelementen modifiziert. Der Aufbau der anorganischen Polymerstruktur kann wie üblich im Rahmen des Sol-Gel Prozesses aus entsprechend funktionalisierten Vorläufersilanen (vgl. Formel (I) bzw. (II)) erfolgen.

Die Vorläufermoleküle sind Silane und die davon abgeleiteten anorganischen Polymere sind Kieselsäurepoly(teil)kondensate, die unter Verwendung von Strukturelementen aufgebaut sind, welche einen teilweise oder vollständig hydrolysierbaren/hydrolysierten und/oder kondensierbaren/kondensierten Silanrest aufweisen. Zusätzlich können diese Kondensate Fremdmetallatome enthalten, die sich in solche Systeme einkondensieren lassen, beispielsweise Bor, Aluminium, Germanium, Zinn, Titan oder Zirkon. Bei fremdmetallhaltigen Kieselsäurepoly(teil)kondensaten handelt es sich dann um Heterokieselsäurepoly(teil)kondensate.

Im Rahmen dieser Anmeldung umfasst der Ausdruck "Vorläufermoleküle oder anorganische Polymere davon" diese Silane und davon abgeleitete Kieselsäurepoly(teil)kondensate sowie Heterokieselsäurepoly(teil)kondensate. Der Ausdruck "Vorläufermoleküle oder anorganische Polymere davon" umfasst die Möglichkeit, dass sowohl Vorläufermoleküle als auch anorganische Polymere davon enthalten sind.

Der mit dem Silanteil verknüpfte organisch polymerisierbare Molekülteil B, der eine oder mehrere Doppelbindungen enthalten kann, kann zum Aufbau eines additiven organischen Netzwerks eingesetzt werden. Die Verbindungseinheit ist organischer Natur und variabel in Länge, Struktur und Zusammensetzung.

Die Silane der Formel (I) bzw. (II) sind über die Reste X hydrolysierbar. Über die hydrolysierbaren Gruppen kann durch Hydrolyse und Kondensation ein ≡Si-O-Si≡ enthaltendes anorganisches Netzwerk aufgebaut werden.

Alle Molekülteile können zur Eigenschaftsmodifikation beitragen. Die zu besonderen Eigenschaftskombinationen führende Variabilität ist durch die eingeführte zusätzliche Funktionalität $R^2$ in variabler Zahl und/oder mit variablem Anteil und variablem Abstand vom Molekülkern gegeben.

Beispiele von Vorläufermolekülen tragen Acrylat- und/oder Methacrylatgruppen als organisch polymerisierbare Molekülteile B.

Wenn hier die Bezeichnung "(Meth)acryl" verwendet wird, so steht diese Bezeichnung sowohl für "Methacryl" als auch für "Acryl". Im Zusammenhang mit Polymeren bedeutet dies, dass das Polymer methacrylfunktionelle Monomere, acrylfunktionelle Monomere oder beide enthält oder aus diesen erhalten wird.

Vorläufermoleküle, die keine Hydroxygruppen tragen, sind beispielsweise ($C_{1-8}$-Alkyl)acrylate und ($C_{1-8}$-Alkyl)methacrylate, insbesondere ($C_{1-4}$-Alkyl)acrylate und ($C_{1-4}$-Alkyl)methacrylate wie Methyl-, Ethyl-, Propyl- und Butyl-(Meth)acrylate. Beispielsweise werden als derartige Vorläufermoleküle Methacrylate eingesetzt, wie Methylmethacrylat und Butylmethacrylat. Die Hydroxygruppen tragenden Vorläufermoleküle enthalten vorzugsweise genau eine Hydroxygruppe pro Monomer. Die Hydroxygruppen können als primäre oder sekundäre Hydroxygruppen vorliegen. Beispielsweise handelt es sich bei den Hydroxygruppen tragenden Vorläufermolekülen um Hydroxyalkyl(meth)acrylate, wie beispielsweise Hydroxy($C_{1-4}$-alkyl)acrylate und/oder Hydroxy($C_{1-4}$-alkyl)methacrylate. Weitere Beispiele sind Hydroxy($C_{1-4}$-alkyl)acrylate wie beispielsweise Hydroxyethylacrylat (HEA) oder Hydroxybutylacrylat (HBA).

In der vorliegenden Anmeldung kann eine mit dem Präfix "Alk" bezeichnete kohlwasserstoffhaltige Gruppe, z. B. in Alkyl, Alkenyl, Alkinyl oder Aralkyl, eine geradkettige oder verzweigte oder cyclische Gruppe sein, solange nichts anderes angegeben ist.

Die Alkylreste in Formel (I) bzw. (II) sind z. B. geradkettige, verzweigte oder cyclische Reste mit 1 bis 20, insbesondere mit 1 bis 10 Kohlenstoffatomen und vorzugsweise niedere Alkylreste mit 1 bis 6, besonders bevorzugt mit 1 bis 4 Kohlenstoffatomen. Spezielle Beispiele sind Methyl, Ethyl, n-Propyl, i-Propyl, n- Butyl, i-Butyl, s-Butyl, t-Butyl, n-Pentyl, n-Hexyl, Cycloh exyl, 2-Ethylhexyl, Dodecyl und Octadecyl.

Die Alkenylreste in Formel (I) bzw. (II) sind z. B. geradkettige, verzweigte oder cyclische Reste mit 2 bis 20, bevorzugt mit 2 bis 10 Kohlenstoffatomen und vorzugsweise niedere Alkenylreste mit 2 bis 6 Kohlenstoffatomen, wie z. B. Vinyl, Allyl und 2-Butenyl.

Bevorzugte Arylreste sind Phenyl, Biphenyl und Naphthyl. Die Alkoxy-, Acyloxy-, Alkylamino- , Dialkylamino-, Alkylcarbonyl-, Alkoxycarbonyl-, Arylalkyl-, Alkylaryl-, Alkylen- und Alkylenarylenreste leiten sich vorzugsweise von den oben genannten Alkyl- und Arylresten ab. Spezielle Beispiele sind Methoxy, Ethoxy, n- und i-Propoxy, n-, i-, s- und t-Butoxy, Monomethylamino, Monoethylamino, Dimethylamino, Diethylamino, N- Ethylanilino, Acetyloxy, Propionyloxy, Methylcarbonyl, Ethylcarbonyl, Methoxycarbonyl, Ethoxycarbonyl, Benzyl, 2- Phenylethyl und Tolyl.

Die genannten Reste können gegebenenfalls einen oder mehrere Substituenten tragen, z. B. Halogen, Alkyl, Hydroxyalkyl, Alkoxy, Aryl, Aryloxy, Alkylcarbonyl, Alkoxycarbonyl, Furfuryl, Tetrahydrofurfuryl, Amino, Monoalkylamino, Dialkylamino, Trialkylammonium, Amido, Hydroxy, Formyl, Carboxy, Mercapto, Cyano, Isocyanato, Nitro, Epoxy, SOsH oder $PO_4H_2$.

Unter den Halogenen sind Fluor, Chlor und Brom und insbesondere Chlor bevorzugt.

Für a ≤ 2 bzw. b=2 können die Reste X und R jeweils dieselbe oder eine unterschiedliche Bedeutung haben.

Der Rest B leitet sich ab von einer substituierten oder unsubstituierten Verbindung $B(AH)_c$ mit mindestens einer C=C- Doppelbindung, wie z. B. Vinyl-, Allyl-, Acryl-, und/oder Methacrylgruppen, und 4 bis 100, vorzugsweise 6 bis 50 Kohlenstoffatomen. Vorzugsweise leitet sich B ab von einer substituierten oder unsubstituierten Verbindung mit zwei

oder mehreren Acrylat- oder Methacrylatgruppen. Derartige Verbindungen werden im Folgenden als (Meth)Acrylate bezeichnet. Falls die Verbindung $B(AH)_c$ substituiert ist, können die Substituenten unter den oben genannten Substituenten ausgewählt sein. Die Gruppe -AH kann -OH, -SH, -NHR, $-NH_2$ oder -COOH sein und c kann Werte von 1 bis 4 einnehmen.

Über seine freien Valenzen ist der Rest B jeweils mit einer Gruppierung $-A(Z)_d-$ verknüpft, die folgende Ausgestaltungen haben kann:
-O-CO-, -S-CO-, -NH-CO-, $-O-CH_2-$, -O-CHR-, $-S-CH_2-$, -S-CHR-, $-NH-CH_2-$, -NH-CHR-, $-CO-O-CH_2-$, -CO-O-CHR-, -O-, -S-, -NH- und -CO-O-, mit R = Alkyl, Aryl oder Alkylaryl. Die Alkylreste sind geradkettige, verzweigte oder cyclische Reste mit 1 bis 20, insbesondere mit 1 bis 10 Kohlenstoffatomen und vorzugsweise niedere Alkylreste mit 1 bis 6, besonders bevorzugt mit 1 bis 4 Kohlenstoffatomen. Spezielle Beispiele sind Methyl, Ethyl, n-Propyl, i-Propyl, n-Butyl, i- Butyl, s-Butyl, t-Butyl, n-Pentyl, n-Hexyl, Cyclohexyl, 2- Ethylhexyl, Dodecyl und Octadecyl. Bevorzugte Arylreste sind Phenyl, Biphenyl und Naphthyl.

Eine in der vorliegenden Erfindung genannte Gruppe "COO" kann Bestandteil sowohl einer Acyloxygruppe (-O-(C=O)-R) als auch einer Alkyloxycarbonylgruppe (-(C=O)-O-R) sein.

Die in der vorliegenden Erfindung eingesetzten Silanen können wie in EP 0 682 033 A2 beschrieben hergestellt werden.

Bei Anwesenheit von zwei oder mehr C=C-Doppelbindungen im Rest B ist die Ausbildung eines dreidimensionalen organischen Netzwerkes möglich. Über den Abstand zwischen dem Si- Atom und dem Rest B, d. h. über die Kettenlänge, und über die Anwesenheit weiterer funktioneller Gruppen in dieser Kette, können die mechanischen Eigenschaften (z. B. Flexibilität) und die physikalisch-chemischen Eigenschaften (Adsorption, Brechzahl oder Haftung) der Poly(hetero)kondensate beeinflusst werden.

In den erfindungsgemäß eingesetzten Silanen haben die Reste und Indices der allgemeinen Formel (I) bzw. (II) vorzugsweise die folgende Bedeutung:

X = $(C_1-C_4)$-Alkoxy, bevorzugt Methoxy und Ethoxy, oder Halogen, bevorzugt Chlor;

R = $(C_1-C_4)$-Alkyl, bevorzugt Methyl und Ethyl;

$R^3$ = $(C_1-C_4)$-Alkylen, bevorzugt Methylen, Ethylen und Propylen;

wobei die anderen Reste und Indices dieselben Bedeutungen wie vorstehend für Formel (I) bzw. (II) definiert haben.

Vorzugsweise stellt B in der allgemeinen Formel (I) bzw. (II) einen substituierten oder unsubstituierten organischen Rest mit einer oder mehreren Acrylat- und/oder Methacrylatgruppen dar. Beispielsweise leitet sich B in Formel (I) bzw. (II) von Acrylsäureestern von Trimethylolpropan, Pentaerythrit, Dipentaerythrit, $C_2$-$C_4$-Alkandiolen, Polyethylenglycolen, Polypropylenglycolen oder, gegebenenfalls substituiertem und/oder alkoxyliertem, Bisphenol A ab.

Funktionalisierung und Derivatisierung

Die erfindungsgemäße 3D-Gerüststruktur ist funktionalisiert, das heißt, sie weist funktionelle Gruppen auf. Diese funktionellen Gruppen werden hier als Funktionalisierungen bezeichnet und sind in Formel (I) bzw. (II) durch $R^2$ dargestellt. $R^2$ ist OH, COOH, $NR^4_2$, $NR^4_2H^+$ oder SH oder ein Derivat davon, wobei $R^4$ Wasserstoff, Alkyl, Aryl oder Alkylaryl ist.

Die erfindungsgemäße 3D-Gerüststruktur ist optional derivatisiert. Das Derivat ist ein Kondensationsderivat von $R^2$, d. h. ein Derivat, das durch Kondensationsreaktion von $R^2$ mit einer anderen Verbindung erhalten werden kann. Genauer gesagt ist das Derivat erhältlich durch Reaktion der Gruppe $R^2$ mit einem nachstehend genannten Biofunktionsmolekül oder Abstandshalter-modifizierten Biofunktionsmolekül. Die Derivatisierung kann vor oder vorzugsweise nach der Photostrukturierung erfolgen.

In $-NR^4_2H^+$ kann die Einschränkung bestehen, dass $R^4$ nicht H ist, außer bei isolierten, nicht "Michael-aktiven" C=C. Ebenso ist in einer Ausführungsform -SH nur bei isolierten, nicht "Michael-aktiven" C=C vorhanden.

Bei Aminofunktionalisierungen ist darauf zu achten, dass die Aktivität nach der Photostrukturierung erhalten bleibt. Es kann daher erforderlich sein, die Amine beispielsweise mit Br-t-BoC zu schützen.

Mittels Derivatisierung kann an die Gruppe $R^2$ eine Verbindung kovalent gebunden werden, welche eine gewünschte biologische Funktion oder Wirkung hat. Sie wird daher in der vorliegenden Erfindung ganz allgemein als "Biofunktionsmolekül" bezeichnet.Anstelle der im Rahmen der vorliegenden Erfindung genannten Biofunktionsmoleküle können auch Farbstoffe als Derivate angebunden werden, so dass die auf Biofunktionsmoleküle bezogene Beschreibung gleichermaßen für Farbstoffe gilt. Als Farbstoff kann beispielsweise Alexa 647 (Alexa 647 Hydrazid, Life Technologies GmbH, Darmstadt, Deutschland) eingesetzt und an die Oberfläche von Carboxy-funktionalisiertem Hybridpolymer angebunden werden. Der Farbstoff hat vorzugsweise ein Molekulargewicht von weniger als 5000, stärker bevorzugt weniger als 2000.

Nach Anspruch 1 ist $R^2$ die Gruppe OH, COOH, $NR^4{}_2$, $NR^4{}_2H^+$ oder SH oder ein Derivat davon. Durch die Derivatisierung entsteht $OR^5$, $COOR^5$, $NR^4R^5$, $NR^4R^5H^+$ oder $SR^5$, wobei $R^5$ der Rest des Biofunktionsmoleküls oder Farbstoffs ist.

Dieses Biofunktionsmolekül kann direkt oder vermittels eines weiter unter beschriebenen Abstandshalters und/oder bifunktionellen Kopplungsmoleküls an $R^2$ gebunden sein.

Somit umfasst ein Derivat eine Verbindung, die ein Kondensat von $R^2$ und Biofunktionsmolekül oder von $R^2$, Abstandshalter und Biofunktionsmolekül ist, wobei jeweils ein bifunktionelles Kopplungsmolekül zwischengeschaltet sein kann.

Beispiele von Biofunktionsmolekülen, die über die funktionelle Gruppe $R^2$ an die erfindungsgemäße 3D-Gerüststruktur gebunden sein können, sind Aminosäuren, Peptide, Proteine, Enzyme, Nukleinsäuren, Nukleotide, Oligonukleotide, Polynukleotide, DNA, RNA, Kohlenhydrate, Monosaccharide, Disaccharide, Oligosaccharide, Polysaccharide, Polymere wie PEG, Antikörper oder Fragmente davon, Kollagen, Fibronektin, Hyaluronsäure, Integrine, und Zellrezeptoren. Bevorzugt ist die Gruppe, die aus Peptiden, Oligonukleotiden und Oligosacchariden besteht, und die Gruppe, die aus Proteinen, Polynukleotiden und Polysacchariden besteht. Die erfindungsgemäße 3D-Gerüststruktur kann eine oder mehrere der genannten Verbindungen enthalten.

Biofunktionsmoleküle können auch Komponenten sein, die eine Zelle in vivo als Mikroumwelt umgeben. Somit kann die erfindungsgemäße 3D-Gerüststruktur eine solche Mikroumwelt simulieren. Eine Mikroumwelt besteht hauptsächlich aus der extrazellulären Matrix (ECM), Wachstumsfaktoren sowie Cytokinen. Die extrazelluläre Matrix enthält Faserproteine, Adhäsionsproteine und Kohlenhydrate. Die Faserproteine können unter Kollagen, Fibrillin oder Elastin ausgewählt sein. Die Kohlenhydrate können unter Glycosaminoglykanen, Proteoglykanen, Hyaluronsäure, Heparansulfat, Dermatansulfat, Chondroitinsulfat und Keratansulfat ausgewählt sein. Die Adhäsionsproteine können unter Lamininen, Vitronektin und Fibronektin ausgewählt sein. Bestandteile der Basalmembran können unter Lamininen, Entaktin und Proteoglycanen ausgewählt sein. Weitere Komponenten sind Zellrezeptoren wie die Integrinfamilie, Peptide und Hydrogele, wie Alginat oder PEG. Die erfindungsgemäße 3D-Gerüststruktur kann eine oder mehrere oder alle der genannten Komponenten der Mikroumwelt einer Zelle enthalten.

Ein bevorzugtes Derivat von $R^2$ enthält mindestens ein Element der Gruppe, die aus einer Aminosäure oder einem Oligomer oder Polymer davon, einem Nukleotid oder Oligomer oder Polymer davon, einem Saccharid oder Oligomer oder Polymer davon, einem Farbstoff und PEG besteht. Ein in dieser Anmeldung genanntes Oligomer enthält mindestens zwei Monomere.

Ein besonders bevorzugtes Derivat von $R^2$ enthält mindestens ein Element der Gruppe, die aus Peptiden, Oligonukleotiden, Oligosacchariden, Proteinen, Polynukleotiden, Polysacchariden, Farbstoffen und PEG besteht. Derivatisierungen von $R^2$ sind mit den funktionellen Gruppen der Biofunktionsmoleküle über die Ausbildung folgender Bindungen möglich:

- OH kann mit COOH oder OH eines Biofunktionsmoleküls eine Ester- bzw. Etherbindung eingehen;
- COOH kann mit OH oder $NH_2$ eines Biofunktionsmoleküls eine Ester- bzw. Amidbindung eingehen;
- $NR^4{}_2$ oder $NR^4{}_2H^+$ kann mit COOH eines Biofunktionsmoleküls eine Amidbindung eingehen;
- SH kann mit COOH, mit OH bzw. SH eines Biofunktionsmoleküls eine Thioesterbindung, Ether- bzw. Thioetherbindung eingehen.

Diese Biofunktionsmoleküle können direkt oder über die weiter unten beschriebenen Abstandshalter (Spacer) an die Gruppe $R^2$ der Gerüststruktur gebunden sein. Wenn ein Abstandshalter eingesetzt wird, kann dieser an einem Ende eine der funktionellen Gruppen enthalten, wie sie vorstehend für die Biofunktionsmoleküle genannt ist, wobei die genannte Bindung zwischen der Gerüststruktur und dem Abstandshalter entsteht. Am anderen Ende kann der Abstandshalter eine der funktionellen Gruppen enthalten, wie sie vorstehend für $R^2$ genannt sind, wobei eine der vorstehend genannten Bindungen entsteht.

Eine Möglichkeit der Ankopplung von Biofunktionsmolekülen oder Farbstoffen oder gegebenenfalls zwischengeschalteten Abstandshaltern besteht über die Verwendung von bifunktionellen Kopplungsmolekülen, die selektiv an funktionelle Gruppen binden und diese wiederum an primäre Aminogruppen koppeln, die den gewünschten Stoff tragen. Beispiele solcher Kopplungsmoleküle sind 1-Ethyl-3-(3-dimethylaminopropyl)carbodiimid-hydrochlorid (EDC, Life Technologies GmbH, Darmstadt, Deutschland) und N-Hydroxysulfosuccinimid (Sulfo-NHS, Life Technologies GmbH, Darmstadt, Deutschland).

Bezüglich $R^2$ in Formel (I) bzw. Formel (II) kann die erfindungsgemäße 3D-Gerüststruktur folgende drei Kombinationen (i) bis (iii), entweder in ein und demselben Gerüststrukturteil oder in unterschiedlichen Gerüststrukturteilen, aufweisen:

(i) H und nicht derivatisiertes $R^2$
(ii) H und nicht derivatisiertes $R^2$ und derivatisiertes $R^2$
(iii) nicht derivatisiertes $R^2$ und derivatisiertes $R^2$.

Neben der vorstehend beschriebenen kovalenten Bindung des Biofunktionsmoleküls entweder direkt oder indirekt über ein Abstandshalter an $R^2$ kann das Biofunktionsmolekül an $R^2$ mit der Funktionalität OH, COOH, $NR^4_2$, $NR^4_2H^+$ oder SH oder einen diese Funktionalität aufweisenden Abstandshalter auch nichtkovalent gebunden werden, beispielsweise über Wasserstoffbrückenbindung, ionische Wechselwirkung oder Komplexierung.

Hierarchischer Aufbau der 3D-Gerüststruktur

In der vorliegenden Erfindung können 3D-Gerüststrukturen oder Strukturteile unterschiedlicher Größenbereiche gebildet werden. Beispielsweise werden Strukturen im Millimeter-, Mikrometer-, Submikrometer- oder Nanometerbereich hergestellt. Dabei bedeutet die Angabe eines Größenwertes einer Struktur, dass in der Struktur Freiräume, Poren oder Hohlräume entstehen, die zumindest in einer Raumrichtung, also in Länge, Breite oder Tiefe, die Abmessungen des angegebenen Wertes aufweisen. Vorzugsweise entsteht eine Struktur mit Poren, deren Ausmaße diesem Größenwert entsprechen. Wenn beispielsweise eine Struktur im Bereich von 1 bis 100 μm gebildet wird, heißt das, dass Poren einer Größe von etwa 1 bis 100 μm entstehen. Entsprechendes gilt für die anderen hier verwendeten Angaben von Größenwerten für Strukturen. Die 3D-Gerüststrukturen können hierarchisch aufgebaut sein und beispielsweise folgende drei Hierarchieebenen aufweisen:

Ebene 1: eine Überstruktur einer Größe von 10 μm bis 100 mm, vorzugsweise 100 μm bis 50 mm, stärker bevorzugt 500 μm bis 5 mm;
Ebene 2: eine Feinstruktur einer Größe von 100 nm bis 1000 μm, vorzugsweise 1000 nm bis 100 μm, stärker bevorzugt 5 μm bis 50 μm;
Ebene 3: Nanopartikel einer Größe von 1 bis 1000 nm, vorzugsweise 5 bis 800 nm, stärker bevorzugt 10 bis 500 nm.

In jedem Fall weist die jeweilige Überstruktur so große Freiräume oder Poren auf, dass eine Vielzahl von gewünschten Feinstrukturen darin gebildet werden kann. Und in jedem Fall weist die jeweilige Feinstruktur so große Freiräume oder Poren auf, dass eine Vielzahl von eingesetzten Nanopartikeln darin enthalten sein kann. Der Ausdruck "Vielzahl" bedeutet mindestens 5, vorzugsweise 10 bis 500. Anders ausgedrückt bedeutet Vielzahl, dass die Struktur in mindestens einer Raumrichtung, vorzugsweise in mindestens zwei Raumrichtungen, also in Längen-, Breiten- oder Tiefenrichtung, ein Ausmaß aufweist, das um den Faktor 5 bis 1000 größer ist als die mittlere Größe der Nanopartikel.

In der erfindungsgemäßen 3D-Gerüststruktur kann die hierarchische Struktur durch mindestens zwei Hierarchiestufen definiert sein. Eine Struktureinheit (I) stellt die übergeordnete Hierarchiestufe dar, Struktureinheit (II) folgt als nächste Hierarchiestufe, und Struktureinheit (III) stellt die unterste der drei Stufen dar. Die Anzahl der Hierarchiestufen ist natürlich nicht auf drei begrenzt. Die durch die Nanopartikel erzeugte zusätzliche Oberfläche stellt eine weitere Hierarchiestufe dar.

In der Ausführungsform (A1) mit mindestens zwei Hierarchiestufen enthält die erfindungsgemäß 3D-Gerüststruktur mindestens eine Struktureinheit (I) einer aus dem Bereich von 10 μm bis 100 mm ausgewählten Dicke (i) und von der Struktureinheit (I) abzweigende Struktureinheiten (II) einer jeweils aus dem Bereich von 100 nm bis 1000 μm ausgewählten Dicke (ii), wobei an den Abzweigungen die Dicke (ii) höchstens die Hälfte der Dicke (i) ist.

In einer Ausführungsform (A2) mit mindestens drei Hierarchiestufen enthält die erfindungsgemäß 3D-Gerüststruktur mindestens eine Struktureinheit (I) einer aus dem Bereich von 100 μm bis 100 mm ausgewählten Dicke (i), von der Struktureinheit (I) abzweigende Struktureinheiten (II) einer jeweils aus dem Bereich von 10 μm bis 1000 μm ausgewählten Dicke (ii) sowie von den Struktureinheiten (II) abzweigende Struktureinheiten (III) einer jeweils aus dem Bereich von 100 nm bis 100 μm ausgewählten Dicke (iii), wobei an den Abzweigungen der Struktureinheiten (II) von der Struktureinheit (I) die Dicke (ii) höchstens die Hälfte der Dicke (i) ist und an den Abzweigungen der Struktureinheiten (III) von den Struktureinheiten (II) die Dicke (iii) höchstens die Hälfte der Dicke (ii) ist.

Die Struktureinheiten (I), (II) und (III) können mit den gleichen oder mit jeweils verschiedenen Strukturierungsverfahren hergestellt werden. Grundsätzlich kann für jede Strukturhierarchiestufe jedes bekannte Strukturierungsverfahren eingesetzt werden.

Beispielsweise können Überstrukturen im Zentimeterbereich durch Rapid-prototyping oder 3D-Druck, Strukturen im Millimeter- bis Mikrometerbereich durch Lithographie und Strukturen im Submikrometerbereich durch MPP realisiert werden.

Nanopartikel

In einer Ausführungsform der vorliegenden Erfindung kann die erfindungsgemäß 3D-Gerüststruktur Nanopartikel enthalten.

Bevorzugt ist der mittlere Durchmesser aller Nanopartikel stets kleiner als die mittlere Dicke der Gerüststruktur. In einer Ausführungsform liegen bevorzugt alle Nanopartikel, bevorzugt überwiegend oder vollständig, in der Gerüststruktur eingebettet vor. Bevorzugt beträgt das Verhältnis der Abmessungen von Nanopartikeln zur Dicke der Gerüststruktur weniger als 1:1, bevorzugt 1:10 oder weniger, weiter bevorzugt 1:100 oder weniger.

Vorzugsweise sind die Nanopartikel in der Gerüststruktur in einem hohen Anteil enthalten. Bevorzugt sind Volumenanteile von 1 bis 60%, stärker bevorzugt 5 bis 30% oder Gewichtsanteile von 1 bis 50%, stärker bevorzugt 5 bis 40%. Die hier genannten Volumenanteile und Gewichtsanteile beziehen sich jeweils auf das Trockengewicht alle Komponenten.

Die Nanopartikel werden durch die Polymerisation des Gerüstmaterials an das entstehende Gerüst gebunden, so dass ein vernetztes Polymer mit daran gebundenen Nanopartikeln erhalten wird. Da Nanopartikel auch an der Grenzfläche von stark und schwach vernetztem Polymer gebunden sind, ragt eine Anzahl von Nanopartikeln zumindest teilweise aus der Struktur heraus.

Die erfindungsgemäße 3D-Gerüststruktur kann Nanopartikel aus anorganischen Substanzen enthalten, zum Beispiel aus Gold oder anderen Edelmetallen oder Metallen oder Metalloxiden, Calciumphosphat und Calciumhydrogensphosphat oder anderen Mischphosphaten, Siliziumbasierten oxidischen Materialien, wie Silicaten, Siliziumoxiden, wie Siliziumdioxid. Die Nanopartikel können auch DynaBeads sein. Die erfindungsgemäße 3D-Gerüststruktur kann Nanopartikel aus organischen Materialien enthalten, insbesondere aus organischen Polymeren. Die organischen Polymere können ausgewählt sein unter Polylactiden (PLA), Poly(lactid-co-glycolid)en (PLGA), Polycaprolactonen (PCL), Polyglycoliden, Di- und Tri-Block-Polymeren bspw. PCL/PGA-Di-Block-Systemen, Polyorthoestern (POE), Polyanhydride, Polyhydroxyalkanoaten (PHA), Polypyrrolen (PPy), Polypropylencarbonat, Polyethylencarbonat, Polyalkylcyanonitril und Polyethylenglycol.

Die erfindungsgemäße 3D-Gerüststruktur kann ein oder mehrere der genannten Nanopartikel aus anorganischen Substanzen und/oder Nanopartikel aus organischen Materialien enthalten.

Die Nanopartikel können zu einer erhöhten Oberflächenrauigkeit führen. Die Oberflächenrauigkeit wird in der vorliegenden Erfindung mittels Rasterkraftmikroskopie bestimmt und als Wert $Z_z$ in Nanometer angegeben. Bevorzugte Werte von Zz sind 10 bis 1000 nm, vorzugsweise 100 bis 800 nm. Alternativ kann die Oberflächenrauigkeit als Wert Zq angegeben werden. Bevorzugte Werte von Zq sind 1 bis 1000 nm, noch stärker bevorzugt 50 bis 100 nm. Die Oberflächenrauigkeit wird bestimmt durch die Größe und die Anzahl oder den Volumenanteil der Nanopartikel in der Struktur. Die bevorzugte Kombination aus mittlerer Größe der Nanopartikel und Volumenanteil der Nanopartikel in der Struktur ist 5 bis 1000 nm bei 3 bis 50%, stärker bevorzugt 30 bis 800 nm bei 10 bis 40%. Dabei ist bei größeren Nanopartikeln der Volumenanteil geringer, so dass es bevorzugt ist, dass das Produkt aus dem in Nanometer angegebenen Zahlenwert der mittleren Größe der Nanopartikel und dem in Prozent angegebenen Zahlenwert des Volumenanteils der Nanopartikel in der Struktur im Bereich von 500 bis 15000 ist.

In der vorliegenden Erfindung wird die Oberflächenrauigkeit nach ISO4287 ermittelt.

Bezüglich der hohen Oberflächenrauigkeit können die Nanopartikel und das photostrukturierbare Material so ausgewählt werden, dass sich die Brechungsindices nur geringfügig unterscheiden. Dadurch ist eine hohe Genauigkeit der Photostrukturierung möglich. Durch die Einstellung der Brechungsindices ist es möglich, eine hohe Konzentration von Nanopartikeln einzusetzen und trotzdem eine hohe Genauigkeit der Photostrukturierung zu erreichen.

Bei der Verwendung von hohlen Partikeln innerhalb einer biodegradierbaren Matrix kann durch die Verwendung von Nährstoffen, Wachstumsfaktoren oder andere Wirkstoffe gezielt das Zellverhalten beeinflusst werden. Dabei wird der Wirkstoff während des Abbaus der Matrix allmählich freigesetzt.

Zur stärkeren und gezielten Bindung der Nanopartikel an die Gerüststruktur können die Nanopartikel und/oder die Strukturmaterialien funktionalisiert sein und über Verknüpfungsgruppen verbunden sein. Diese Verknüpfungsgruppen können als Abstandshalter dienen. Wenn diese Abstandshalter unterschiedlich lang sind, kann die effektive Oberfläche der Struktur zusätzlich erhöht werden. In einer Ausführungsform kann der Einbau der Nanopartikel in die Struktur und die Verknüpfung mit Nanopartikeln mittels Funktionalisierung mit ggf. unterschiedlich langen Abstandshaltern kombiniert werden. Die Bindung der Nanopartikel kann über die funktionelle Gruppe $R^2$ in Formel (I) bzw. (II) oder über eine andere Funktionalisierung erfolgen. Durch Anbindung der Nanopartikel an einen Teil des Vorläufermoleküls der Formel (I) bzw. (II) kann der Gehalt an Nanopartikel eingestellt werden.

Die Oberfläche der Nanopartikel kann chemisch funktionalisiert werden, um den Zellen eine geeignete chemische Grenzfläche zu schaffen oder auch um die Nanopartikel kovalent über entsprechende photovernetzbare Gruppen (z.B. 3-(Trimethoxysilyl)propylmethacrylat) an das Matrixmaterial zu binden. Die Oberfläche der Nanopartikel kann auch mit bioaktiven Materialien bzw. Molekülen funktionalisiert werden wie sie vorstehend als Biofunktionsmoleküle beispielhaft genannt sind.

Darüber hinaus können die Nanopartikel mesoporös ausgeführt und mit entsprechenden Wirkstoffen, Medikamenten, Nährlösung oder ähnlichem gefüllt sein (Drug-delivery Prinzip).

Dabei kann die reaktive Gruppe eines Nanopartikels mit der reaktiven Gruppe eines Biofunktionsmoleküls eine kovalente Bindung eingehen. Das Nanopartikel weist also auf seiner Oberfläche eine erste funktionelle Gruppe 1A auf, die kovalent mit einer funktionellen Gruppe 2A des Biofunktionsmoleküls verknüpft ist, wobei die funktionelle Gruppe 1A eine andere Gruppe als die funktionelle Gruppe 2A ist. Die beiden miteinander in Bindung tretenden Gruppen 1A und 2A müssen komplementär zueinander sein, das heißt in der Lage sein, eine kovalente Bindung miteinander einzugehen. Es können die gleichen Gruppen 1A und 2A wie weiter unten für die Bindung des Biofunktionsmoleküls an den Abstandshalter eingesetzt werden.

Die Nanopartikel können alternativ oder zusätzlich zu der genannten Oberflächenfunktionalisierung im Inneren Nährstoffe, Botenstoffe, Zellrezeptoren oder andere Stoffe enthalten, die in ein Medium ggf. verzögert freigesetzt werden können. Die Nanopartikel können einen oder mehrere dieser Stoffe enthalten, die unter Wachstumsfaktoren, Cytokinen, Zelladhäsionsproteinen, Farbstoffen wie Fluorescenaminen, Chemokinen, Vitaminen, Mineralstoffen, Fetten, Proteinen, Nährstoffen, faserbildende Proteinen, Kohlenhydraten, Adhäsionsproteinen, Zellrezeptoren, Pharmazeutika, DNA, RNA, Aptameren, angiogenen Faktoren, Lektinen, Antikörpern, Antikörperfragmenten, Peptiden, Hydrogelen oder Inhibitoren ausgewählt sind.

Die Nanopartikel können Stabilisatoren aufweisen, die unter Kohlenhydraten, zum Beispiel Trehalosen, Proteinen, Polyethylenglycolen oder Detergentien ausgewählt sind.

In einer Ausführungsform können die Nanopartikel ein oder mehrere der Biofunktionsmoleküle enthalten, die vorstehend als Komponenten der Mikroumwelt von Zellen beschrieben wurden.

Diese Biofunktionsmoleküle können als Derivatisierungen von $R^2$ an die 3D-Gerüststruktur direkt kovalent gebunden sein und/oder an die Nanopartikel kovalent gebunden sein und/oder in den Nanopartikeln nicht-kovalent eingebettet enthalten sein.

Abstandshalter

Ein Biofunktionsmolekül kann über einen Abstandshalter (Spacer) an die Gruppe $R^2$ gebunden sein.

Der Abstandshalter kann vor oder vorzugsweise nach der Photostrukturierung angebunden werden.

Der Abstandshalter ist mindestens bifunktionell, d. h., er weist eine reaktive Gruppe auf, die mit der Gruppe $R^2$ der erfindungsgemäßen 3D-Gerüststruktur vorzugsweise in einer Kondensationsreaktion umgesetzt wird, und er weist eine reaktive Gruppe auf, die mit einer reaktiven Gruppe des Biofunktionsmoleküls vorzugsweise in einer Kondensationsreaktion umgesetzt wird. Vorzugsweise reagieren beide reaktiven Gruppen in einer Kondensationsreaktion.

Die beiden reaktiven Gruppen des Abstandshalters können gleich oder verschieden sein.

Wenn sie verschieden sind, kann die mit dem Biofunktionsmolekül umzusetzende reaktive Gruppe des Abstandshalters auch eine erste funktionelle Gruppe 1A sein, die mit einer komplementären Gruppe 2A eines Biofunktionsmoleküls eine affine, bevorzugt kovalente Bindung eingehen kann. Die erste funktionelle Gruppe 1A kann ausgewählt sein aus der Gruppe bestehend aus Aminogruppe, Carboxygruppe, Epoxygruppe, Maleinimidogruppe, Alkylketongruppe, Aldehydgruppe, Hydrazingruppe, Hydrazidgruppe, Thiolgruppe und Thioestergruppe. Die funktionelle Gruppe 2A des Wirkstoffs kann ausgewählt sein aus der Gruppe bestehend aus Aminogruppe, Carboxygruppe, Epoxygruppe, Maleinimidogruppe, Alkylketongruppe, Aldehydgruppe, Hydrazingruppe, Hydrazidgruppe, Thiolgruppe und Thioestergruppe. Ein Abstandshalter weist also auf seiner Oberfläche eine erste funktionelle Gruppe 1A auf, die kovalent mit einer funktionellen Gruppe 2A des Biofunktionsmoleküls verknüpft ist, wobei die funktionelle Gruppe 1A eine andere Gruppe als die funktionelle Gruppe 2A ist. Die beiden miteinander in Bindung tretenden Gruppen 1A und 2A müssen komplementär zueinander sein, das heißt in der Lage sein, eine kovalente Bindung miteinander einzugehen.

Der Abstandshalter weist vorzugsweise eine optional durch Sauerstoffatome und/oder Aminogruppen unterbrochene Kohlenwasserstoffkette mit einer Kettenlänge von vorzugsweise von 5 bis 10.000 Atomen auf.

Beispielsweise kann der Abstandshalter über Aminogruppen an das 3D-Gerüststruktur gebunden sein, wenn $R^2$ eine Carbonsäuregruppe ist. Insbesondere kann der Abstandshalter ein mindestens difunktionelles Amine gegebenenfalls unterschiedlicher Länge wie Polyethylenglycol-$NH_2$ und/oder eines Polyalkylenamins wie z.B. Tetraethylenpentamin sein.

In diesem Zusammenhang sollen die Ausdrücke "von Polyalkylenglycol-$NH_2$ abgeleitet", "von Polyethylenglycol-$NH_2$ abgeleitet", "von Polyalkylenamin abgeleitet" und "von Tetraethylenpentamin abgeleitet" bedeuten, dass ein endständig mit $NH_2$-Gruppen modifiziertes Polyalkylenglycol/Polyethylenglycol bzw. ein Oligoalkylenamin/Tetraethylenpentamin verwendet wird, so dass der entstehende Abstandshalter mindestens zwei Kopplungsgruppen aufweist, wobei über eine dieser Gruppen die erfindungsgemäße 3D-Gerüststruktur und über die andere dieser Gruppen das Biofunktionsmolekül gebunden ist. Die Bindung ist in der Regel jeweils als -C(O)NH-Kopplungsgruppe ausgestaltet.

Der Abstandshalter umfasst vorzugsweise sowohl Abstandshalter, die von Polyethylenglycol-$NH_2$ unterschiedlicher Länge abgeleitet sind, als auch Abstandshalter, die von Tetraethylenpentamin abgeleitet sind.

Durch die Auswahl der Gruppe $R^2$ der Gerüststruktur, der Gruppen an beiden Enden des Abstandshalters und der reaktiven Gruppe des Biofunktionsmoleküls kann eine Verknüpfung 3D-Gerüststruktur/Abstandshalter/Biofunktionsmolekül unter Ausbildung der vorstehend für die Derivatisierung beschriebenen Bindungen erhalten werden.

Die Anbindung des Biofunktionsmoleküls erfolgt beispielsweise durch Umsetzen einer Carboxygruppe des Biofunktionsmoleküls, z. B. dem C-terminalen Enden eines Peptids, mit einer Aminogruppe des Abstandshalters. Durch diese Umsetzung entstehen Amidgruppen.

Vorzugsweise ist also der Abstandshalter über Amidgruppen auch an die erfindungsgemäße 3D-Gerüststruktur gebunden. Diese Umsetzung kann z.B. mittels eines im Stand der Technik bekannten Verfahrens unter Einsatz einer Carbodiimidverbindung als Katalysator erfolgen.

In einer Ausführungsform sind eventuell vorhandene freie funktionelle Gruppen der 3D-Gerüststruktur und/oder des Abstandshalters blockiert.

Die kovalente Bindung ist beispielsweise erhältlich zwischen dem Abstandshalter und dem Biofunktionsmolekül durch Umsetzen einer Carboxylgruppe des Biofunktionsmoleküls mit einer Aminogruppe des Abstandshalters oder durch Umsetzen einer mittels Azidoacetylchlorid azidierten Aminogruppe mit einem DNA- oder RNA-Molekül erhältlich ist.

Durch das Vorhandensein eines Oberflächenabstandhalters variabler Länge wie voranstehend beschrieben, insbesondere unterschiedlicher Länge, wird die Mobilität des Biofunktionsmoleküls erhöht und dadurch die Reaktionskinetik mit anderen Verbindungen verbessert.

Der Abstandshalter besitzt vorzugsweise endseitig jeweils Aminogruppen. Eine dieser Gruppen wird zur Anbindung an die 3D-Gerüststruktur genutzt, die andere dient zur kovalenten Bindung an das Biofunktionsmolekül. Damit wird es möglich, Biofunktionsmoleküle auf günstige und gängige Weise anzubinden.

Durch die Verwendung von Abstandshaltern unterschiedlicher Länge kann die Anzahl der Biofunktionsmoleküle pro Flächeneinheit oder Volumeneinheit deutlich erhöht werden, so dass die gewünschte Biofunktion verstärkt werden kann.

Die erfindungsgemäße 3D-Gerüststruktur kann vor dem Anbinden des Biofunktionsmoleküls mit dem Abstandshalter modifiziert werden.

Das freie Amino-Ende der Funktionalisierung des PEG-$NH_2$ oder TEPA eignet sich zum Anbinden an ein Biofunktionsmolekül.

Proteine können über kovalente Bindungen an COOH-Gruppen der 3D-Gerüststruktur angebunden werden. Da Proteine aus Aminosäuren aufgebaut sind, besitzen sie sowohl freie -COOH als auch freie -$NH_2$-Gruppen. Deswegen sind sowohl Carboxy- als auch Aminreste an der 3D-Gerüststruktur als Bindungsterminal zum Protein möglich, um eine Amidbindung zu bilden.

Tetraethylenpentamin (TEPA) der Formel $H_2N\text{-}CH_2CH_2\text{-}NH\text{-}CH_2CH_2\text{-}NH\text{-}CH_2CH_2\text{-}NH\text{-}CH_2CH_2\text{-}NH_2$ kann ein Gemisch aus mehreren Verbindungen (Oligomeren) sein, die aus einer unterschiedlichen Monomerzahl entstanden sind und daher eine unterschiedliche Kettenlänge aufweisen. Das in der vorliegenden Verbindung verwendete TEPA kann daher z.B. eine oder mehrere der Verbindungen enthalten, die aus der Gruppe ausgewählt sind, die aus N-(2-Aminoethyl)-N'-{2-{2-aminoethyl)amino}ethyl}-1,2-ethandiamin; 4-(2-Aminoethyl)-N-(2-aminoethyl)-N'-{2-{(2-aminoethyl)amino}ethyl}-1,2-ethandiamin; 1-(2-Aminoethyl)-4-[(2-aminoethyl)amino]ethyl]piperazin) und 1-[2-[[2-[(2-Aminoethyl)amino]ethyl]amino]ethyl]piperazin) besteht. Diese Verbindungen unterscheiden sich in ihrer Struktur und damit ihrer Reaktivität mit den Reaktionspartnern, nämlich der erfindungsgemäßen 3D-Gerüststruktur einerseits und dem Biofunktionsmolekül andererseits. Somit umfasst der hier verwendete Ausdruck "Tetraethylenpentamin" oder "TEPA" nicht nur die Verbindung der vorstehenden Formel, sondern auch jede der anderen vorstehend genannten Verbindung allein oder in einem Gemisch von zwei oder mehreren dieser Verbindungen.

Das in der vorliegenden Erfindung verwendete Polyethylenglycol-$NH_2$(PEG-$NH_2$) leitet sich von Polyethylenglycol (PEG) ab. PEG ist ein Polymer aus Ethylenglycol-Monomereinheiten und kann unterschiedlichste Kettenlängen aufweisen. Typische Kettenlängen von käuflich erwerbbarem PEG umfassen 200, 400 oder 600 Monomereinheiten. In der vorliegenden Erfindung kann das PEG-$NH_2$ vorzugsweise aus 5 bis 600 der Monomereinheiten bestehen. Es kann PEG-$NH_2$ einer einzigen Kettenlänge oder PEG-$NH_2$ verschiedener Kettenlängen eingesetzt werden. Das heißt, die vorliegende Erfindung umfasst auch eine Ausführungsform, in der ein Gemisch aus PEG-$NH_2$ verschiedener Kettenlänger eingesetzt wird. Zusätzlich kann dieses Gemisch auch ein oder mehrere der vorstehend genannten verschiedenen TEPAs enthalten.

Auf diese Weise können Abstandshalter verschiedener Länge und auch Reaktivität auf der erfindungsgemäßen 3D-Gerüststruktur erhalten werden. Die Verwendung von Abstandshaltern verschiedener Länge hat den Vorteil, dass die sensorische Oberfläche erhöht werden kann. Konkret bedeutet dies, dass auf der 3D-Gerüststruktur die Biofunktionsmoleküle quasi in mehreren Schichten und trotzdem gut zugänglich vorhanden sein können.

In einer Ausführungsform der vorliegenden Erfindung können die $NH_2$-Termini der Gruppen $R^2$ mittels Azidoacetylchlorid azidiert werden. Durch diese Funktionalisierung kann Klick-Chemie als Bindungsmechanismus zwischen der 3D-Gerüststruktur und dem Biofunktionsmolekül genutzt werden. Über die Klick-Chemie können die verschiedensten Moleküle an die 3D-Gerüststruktur gebunden werden. Dies kann ohne die Zugabe zusätzlicher Reaktionschemie erfolgen.

Verwendung der erfindungsgemäßen 3D-Gerüststruktur

Die erfindungsgemäße 3D-Gerüststruktur kann für die Wechselwirkung mit biologischen Zellen in vitro oder in vivo eingesetzt werden.

Bei der medizinischen Verwendung im oder am menschlichen oder tierischen Körper betrifft die vorliegende Erfindung eine 3D-Gerüststruktur für die medizinische, nämlich therapeutische oder diagnostische, Verwendung. Diese Verwendung beruht auf der Wechselwirkung mit biologischen Zellen.

Beim Einsatz für 3D-Zellkulturen ermöglichen photostrukturierbare ORMOCER®e hochaufgelöste 3D-Zellgerüststrukturen, die genau auf die Bedürfnisse verschiedener Zelltypen angepasst sind. Durch geeignete Topographien (Dimensionalität, Krümmung, Porengröße, Rauheit), sowie chemische und physikalische Materialeigenschaften lassen sich realistische, aber genau kontrollierbare Wachstumsbedingungen schaffen.

Beim Einsatz der 3D-Gerüststrukturen für Implantate verbessert die Wahl geeigneter Oberflächen- und Materialeigenschaften die Biokompatibilität von Implantaten. Räumlich variierende Scaffold-Eigenschaften können die Ansiedelung verschiedener Zelltypen an unterschiedlichen Stellen des Implantats fördern und unterstützen damit seine korrekte Funktionsweise und Integration im Körper.

Beim Einsatz für Diagnosemethoden (cell sorting, lab-on-a-chip) kann die Affinität der erfindungsgemäßen 3D-Gerüststrukturen zu verschiedenen Funktionalisierungen dazu genutzt werden, unterschiedliche Zelltypen voneinander zu trennen (z.B. gesund und pathogen), indem diese dazu angeregt werden, in verschiedene Richtungen zu migrieren. Außerdem ist eine ortsselektive Adhäsion von bestimmten Zelltypen möglich. In Verbindung mit hochauflösender Mikrostrukturierung ist dies vor allem für Lab-on-a-chip-Anwendungen interessant.

Beim Einsatz der 3D-Gerüststrukturen für das Tissue Engineering müssen für die Züchtung komplexer Gewebe im Labor unterschiedliche Zelltypen dazu gebracht werden, verschiedene Stellen der Gerüststrukturen zu besiedeln und dort im Zusammenspiel mit anderen Zellen ihre Funktion auszuüben. Diese Bereiche können in einem selektiven scaffold aus verschiedenen Materialien jeweils unabhängig voneinander auf den gewünschten Zelltyp und seine beabsichtigte Funktion abgestimmt werden.

Verfahren zur Herstellung der erfindungsgemäßen 3D-Gerüststruktur

Eine Möglichkeit zur Herstellung feinstrukturierter Gerüste bzw. hochkomplexer Strukturen bietet die Stereolithographie. Bei dieser Technik wird eine chemische Reaktion, nämlich Photopolymerisation, durch elektromagnetische Strahlung initiiert. Das zu strukturierende Material beziehungsweise die zu strukturierende Materialformulierung muss folglich die Anforderung erfüllen, mit Licht reagieren zu können. Dadurch findet in den exponierten Bereichen ein Phasenübergang von flüssig zu fest statt. Im nachfolgenden Entwicklungsschritt wird somit nur das verbliebene flüssige Material weggelöst. Mit Hilfe eines Laserstrahls können so schichtweise Strukturen erzeugt werden. Vorteile dieser Methode sind, dass hochorganisierte dreidimensionale Gerüststrukturen mit definierter Porosität, Porengröße und Interkonnektivität der Poren mit hoher Reproduzierbarkeit hergestellt werden können und dies mit nur wenigen Prozessschritten möglich ist.

Eine gute Auflösung bietet ein Spezialfall der Stereolithographie, nämlich die Zwei-Photonen-Polymerisation (two photon polymerization, 2PP). Mit dieser Technik können ultrafeine Strukturen bei hoher Auflösung erzeugt werden. Sie beruht auf der simultanen Absorption von zwei Photonen (two photon absorption, TPA), die den Zerfall der Initiatormoleküle und somit auch die nachfolgende chemische Reaktion zwischen den erzeugten Radikalen und den Monomeren auslöst. Für die Anregung sind aufgrund der simultanen Absorption von zwei Photonen sehr hohe Lichtintensitäten nötig, die durch ultrakurze Laserpulse realisiert werden können. Die Rate der Zwei-Photonen-Absorption hängt dabei nichtlinear von der Intensität ab.

Infolgedessen findet die Polymerisation nur in einem räumlich eng begrenzten Bereich um den Laserfokus herum innerhalb der zu verfestigenden Flüssigkeit statt, wodurch auch Auflösungen von < 100 nm erreicht werden können. Wird der Fokus durch das Material bewegt, werden in den belichteten Bereichen in einem Prozessschritt dreidimensionale Mikrostrukturen erzeugt. Damit lässt sich mit relativ guter Genauigkeit ein nur kleiner Raum innerhalb der zu verfestigenden Flüssigkeit ansteuern, der durch die eingetragene Energie verfestigt wird. Die Herstellung des Formkörpers kann daher innerhalb einer entsprechenden Flüssigkeit erfolgen, nicht mehr (nur) an deren Oberfläche.

In einer bevorzugten Ausgestaltung der Erfindung wird die Flüssigkeit mittels Bestrahlung mit Femtosekunden-Laserpulsen verfestigt. Dabei können übliche polymerisierbare Materialien ultrakurzen Laserpulsen mit hoher Spitzenenergie ausgesetzt werden, z. B. Ti:Saphir-Femtosekundenlaser-Pulsen. Diese werden mit Wellenlängen im Bereich von etwa 800 nm in die zu verfestigenden Harze eingestrahlt.

Als hierfür geeignete Laser lassen sich vorzugsweise Ti-Saphir-Laser einsetzen (entweder mit der fundamentalen Wellenlänge von 780 nm oder, je nach Absorptionsverhalten der zu erhärtenden Flüssigkeit, mit der zweiten

Harmonischen bei 390 nm); auch andere NIR-Laser (z.B. mit emittierten Wellenlängen von 800 nm bis etwa 1500 nm) sind geeignet. Aber auch andere Laserbestrahlung ist möglich, sofern die eingesetzte Lichtquelle mit einer Intensität in die Flüssigkeit einstrahlen kann, die für eine Mehrphotonenanregung geeignet ist. Diese Eigenschaft bieten bei moderaten mittleren Leistungen insbesondere Kurzpulslaser. Das auszuhärtende Material muss transparent für die verwendete Laserwellenlänge sein. Wenn das zu verfestigende Material beispielsweise bei 390 nm einer Einphotonen-Polymerisation zugänglich wäre, kann jede Wellenlänge von 400 nm oder darüber für die Zwei- oder Mehrphotonenpolymerisation eingesetzt werden; je nach Harz sind wegen der Transparenzbedingungen vor allem 500-1000 nm geeignet. Bei Verwendung größerer Wellenlängen kann die Polymerisation auch durch eine n-Photonenabsorption initialisiert werden, wobei n größer 2 gilt.

Insbesondere bei Einsatz von Femtosekundenlasern als Strahlungsquelle erhält man Körper/Schichten mit hoher lithographischer Auflösung. Art und Dauer der Bestrahlung liefern darüber hinaus die Möglichkeit, den Vernetzungsgrad gezielt zu variieren, so dass mit ein und demselben Material bei Bedarf unterschiedliche physikalische Eigenschaften (z.B. unterschiedlich schnelle Abbaubarkeit, einstellbarer E-Modul) erzielt werden können. Die Variation des Vernetzungsgrades kann auch innerhalb einer Gerüststruktur erfolgen. Dies kann entweder an bestimmten Stellen ausgeführt sein oder als Gradient vorliegen. Mit diesem Verfahren können nicht nur dreidimensionale Strukturen auf Substraten (Trägermaterialien) gefertigt werden, sondern es können dreidimensionale freitragende Körper vollständig aus dem Volumen heraus gefertigt werden.

Durch das erfindungsgemäße Verfahren ist es möglich, dreidimensionale Formkörper mit einem durchgängigen porösen Netzwerk innerhalb des Formkörpers großflächig durch Lichtinduzierte Vernetzungsprozesse, insbesondere durch die Mehr-Photonen-Absorptions-Technologie über einen weiten Wellenlängenbereich unter Verwendung verschiedenster Laser- und optischer Systeme in-situ herzustellen. Insbesondere lassen sich mit dem beschriebenen Verfahren große Strukturen und Formkörper mit einer Größe bis in den cm-Bereich herstellen.

Durch die Kombination verschiedener photostrukturierender Verfahren können gewünschte hierarchische Strukturen aufgebaut werden. Auf diese Weise ist eine hierarchische Struktur vom Zentimeter- bis zum Nanometerbereich beliebig anpassbar. Beispielsweise können Überstrukturen im Zentimeterbereich durch ein Rapid-prototyping-Verfahren und Unterstrukturen durch Mehr-Photonen-Absorption (MPA) hergestellt werden.

Die Porenstrukturen werden nach Wunsch ausgewählt, z. B. in Abhängigkeit von den Zelltypen, die auf die Trägermatrix aufzubringen sind.

Im Gegensatz zu den stereolithographischen Verfahren, welche einen Gegenstand schichtweise in einem Bad einer durch Strahlungseinwirkung verfestigbaren Flüssigkeit unter Bewegung des Objektes weiter in das Bad hinein verfestigen, wird bei der Erzeugung mittels Femtosekundenlaser-Bestrahlung ein zu erzeugender dreidimensionaler Körper also in nur einem Arbeitsschritt auf einer Oberfläche oder im Volumen produziert.

Die Zwei- oder Mehrphotonenpolymerisation des organischen, über Zwei-Photonen- oder Mehrphotonen-Polymerisation polymerisierbaren Rests kann über eine bzw. mehrere Gruppen erfolgen, die sich radikalisch polymerisieren lassen. Als radikalisch polymerisierbare Gruppen eignen sich nichtaromatische C=C-Doppelbindungen wie Allyl- oder Vinylgruppen, besonders bevorzugt sind allerdings einer Michael-Addition zugängliche Doppelbindungen.

Beispiele von photostrukturierbaren Matrixmaterialien sind anorganisch kondensierbare Silane und deren Kondensate und/oder Polymerisate. Diese Silane enthalten eine(n) oder mehrere über Sauerstoff an das Silicium gebundene Substituenten bzw. Gruppen mit Ester-, ggf. darüber hinaus mit Ether- und/oder Thioetherbindungen, sowie organisch polymerisierbare Einheiten wie C=C-Doppelbindungen oder ringöffnende Systeme, z.B. Epoxygruppen. Die Silane lassen sich anorganisch kondensieren und/oder organisch über die C=C-Doppelbindungen polymerisieren. Eine solche organische Polymerisation kann beispielsweise mit Hilfe von 2- Photonen-Polymerisation (2PP) erfolgen, so dass sich strukturierte, im Wesentlichen monomerfreie Materialien erhalten lassen. Durch Variation des Anteils an anorganisch vernetzbaren Einheiten und/oder an organisch polymerisierbaren Einheiten lassen sich die mechanischen Eigenschaften der aus den Silanen erzeugten Hybridpolymere gezielt so einstellen, dass sie denjenigen natürlicher, weicher oder harter Gewebe ähneln. Diese Hybridpolymere sind besonders geeignet zur Herstellung von Gerüststrukturen.

Die bekannten ORMOCER®-Hybridmaterialien lassen sich sowohl über eine organische Polymerisation von C=C-Doppelbindungen als auch über anorganische Vernetzungsreaktionen (Si-O-Si-Brücken-Bildung) verfestigen, wobei das Vorhandensein von organisch polymerisierbaren C=C-Doppelbindung eine räumliche Strukturierung bei der Polymerisation ermöglicht.

In einer Ausführungsform der Erfindung wird eine gewünschte dreidimensionale Matrixstruktur hergestellt.

Dabei werden in einem ersten Schritt des Verfahrens zur Herstellung einer gewünschten Struktur Daten bereitgestellt, welche den gewünschten strukturellen Aufbau beschreiben. Durch die Daten wird die geometrische Form der Struktur beschrieben.

In einem weiteren Schritt des Verfahrens wird ein Vorläufer eines Biopolymers bereitgestellt. Bei dem Vorläufer handelt es sich um einen Ausgangsstoff im Sinne eines Präkursors des Biopolymers, beispielsweise in Form eines Monomers. Bei dem Biopolymer handelt es sich beispielsweise um ein biokompatibles und/oder bioabbaubares Polymer.

Es erfolgt ein örtlich gezieltes Bestrahlen des Vorläufers mit einer elektromagnetischen Strahlung, wobei das

Bestrahlen, insbesondere die Auswahl der zu bestrahlenden Bereiche, gemäß dem durch die Daten beschriebenen strukturellen Aufbau erfolgt. Es werden also genau diejenigen zusammenhängenden Teilmengen der Ebene bzw. des Raumes bestrahlt, welche durch die Daten definiert sind. Die elektromagnetische Strahlung kann beispielsweise durch ein fokussiertes Licht oder durch einen Laserstrahl gebildet sein.

Erfindungsgemäß wird die elektromagnetische Strahlung so bemessen, dass in den bestrahlten Bereichen des Vorläufers eine Zwei- oder Multiphotonen-Absorption stattfindet, durch welche der Vorläufer in den bestrahlten Bereichen zu dem Biopolymer polymerisiert wird, sodass er dort zumindest teilweise verfestigt. Da das Bestrahlen des Vorläufers örtlich gezielt entsprechend dem durch die Daten beschriebenen strukturellen Aufbau erfolgt, wird das gebildete Polymer mit der gewünschten Struktur ausgebildet.

In einer weiteren Ausführungsform des Verfahrens werden Anteile des Vorläufers, welche nach dem Bestrahlen nicht polymerisiert sind, ausgeschwemmt. Durch das Ausschwemmen werden u. a. Hohlräume entleert.

Das Verfahren zur Herstellung der erfindungsgemäßen 3D-Gerüststruktur umfasst folgende Schritte:

(a) Bereitstellen von photopolymerisierbaren Monomeren mit einer Funktionalisierung,
(b) Photostrukturierung der in Schritt (a) bereitgestellten Monomere unter Bildung einer 3D-Gerüststruktur,
(c) Entfernen der verbleibenden Monomere.

In dem erfindungsgemäßen Verfahren sind außerdem die folgenden Aspekte (i) bis (iv) von Bedeutung, wobei (i) die Zusammensetzung der Monomeren in Schritt (a), (ii) die Wiederholung der Schritte (a) bis (c), (iii) die Derivatisierung der Funktionalisierung und (iv) die Reihenfolge der Verfahrensschritte betrifft:

(i) Durch die Schritte (a) bis (c) können Polymere hergestellt werden, in denen jede Polymerkette eine einzige oder mehrere Funktionalisierungsarten aufweist. Wenn beispielsweise ein Homopolymer aus einem eine Funktionalisierung aufweisenden Monomer hergestellt wird, enthält das Homopolymer nur eine einzige Funktionalisierungsart. Wenn beispielsweise ein Copolymer aus einem Monomer mit einer Funktionalisierung und einem Monomer mit einer anderen Funktionalisierung hergestellt wird, weist das Copolymer zwei unterschiedliche Funktionalisierungsarten auf. Wenn beispielsweise ein Copolymer aus einem Monomer mit einer Funktionalisierung und einem Monomer ohne Funktionalisierung, d. h. $R^2$ = H in Formel (I) bzw. (II), hergestellt wird, weist das Copolymer einen geringeren Funktionalisierungsgrad auf. Beispielsweise kann durch den Einsatz von Gemischen aus Monomeren, Oligomeren oder Präpolymeren unterschiedlicher Zusammensetzung Polymere erhalten werden, welche die gewünschten Funktionalisierungen in der gewünschten Anzahl und in den gewünschten Polymerkettenbereichen aufweisen.
Ein weiteres Beispiel ist bei der Funktionalisierung $R^2$ = SH denkbar. Durch die Variation von Index c kann das Verhältnis der C=C-Doppelbindungen zu $R^2$ = SH beliebig eingestellt werden. Bei Durchführung einer Thiol-En-Polymerisation können die SH-Gruppen im Überschuss zu den C=C-Doppelbindungen eingesetzt werden, so dass schließlich nicht umgesetzte SH-Gruppen übrigbleiben.

(ii) Die Schritte (a) bis (c) können unter Verwendung anderer Monomere wiederholt werden, wodurch 3D-Gerüststrukturen mit einer Funktionalisierung in einem Gerüststrukturteil und einer strukturell oder zahlenmäßig anderen Funktionalisierung in einem anderen Gerüststrukturteil gezielt erhalten werden können. In der vorliegenden Erfindung entspricht das Mindestmaß eines Gerüststrukturteils in jeder Raumrichtung der Auflösung bei der Photostrukturierung, nämlich 100 nm, wobei ein Mindestmaß von 1 $\mu$m bevorzugt ist.

(iii) Die funktionalisierten Monomere können mit anderen Verbindungen umgesetzt und damit derivatisiert werden. Beispielsweise kann eine Hydroxyfunktionalisierung mit einer Carbonsäure zu einem Ester umgesetzt werden. An dieser Carbonsäure kann ein Molekül beliebiger Funktion hängen, beispielsweise eine Gruppe mit biologischer Affinität oder Funktion. Die Derivatisierung der Hydroxygruppe kann bereits am Monomer erfolgen, so dass das Derivat gleichmäßig in die erfindungsgemäße 3D-Gerüststruktur eingebaut wird und somit durchgehend, nämlich an der Oberfläche und im Inneren, in einem Strukturteil vorhanden ist. Die Derivatisierung kann aber auch nach erfolgter Strukturierung oder nach erfolgter Teilstrukturierung (vgl. den vorstehenden Aspekt (ii)) durchgeführt werden. Dadurch können gewünschte Gruppen eingeführt werden, wobei diese Gruppen hauptsächlich an die Funktionalisierungen auf den Oberflächen der 3D-Gerüststruktur oder den Oberflächen der 3D-Gerüststrukturteile und weniger an die Funktionalisierungen in engmaschigeren Inneren der Struktur binden. Auf diese Weise können gezielt 3D-Gerüststruktur hergestellt werden, in denen sich die Funktionalitäten an der Oberfläche von den Funktionalitäten im Inneren des strukturierten Elements qualitativ oder quantitativ unterscheiden.

(iv) Bei der Herstellung der erfindungsgemäßen 3D-Gerüststruktur aus einem Ausgangsmaterial, das ein Vorläufermolekül enthält, können grundsätzlich folgende Verfahren zum Einsatz kommen: (1) anorganische Polymerisierung (Hydrolyse und Kondensation) des Vorläufermoleküls; (2) Derivatisierung von $R^2$; (3) Photostrukturierung.

Diese Verfahren können in den alternativen Reihenfolgen 1-2-3, 2-1-3, 1-3-2 ausgeführt werden.

Wird zusätzlich ein Verfahren (4) der Einführung eines Abstandshalters durchgeführt, sind die alternativen Reihenfolgen 1-4-2-3, 4-2-1-3, 1-4-3-2, 1-4-3-2, 4-1-2-3, 4-1-3-2 möglich.

Durch eine gezielte Kombination von (i) bis (iv) können 3D-Gerüststrukturen erhalten werden, in denen Strukturteile enthalten sind, die unabhängig voneinander eine oder mehrere gewünschte Funktionalisierungsarten aufweisen, worin zusätzlich in jedem Strukturteil ein Funktionalisierungsderivat entweder durchgehend oder hauptsächlich an der Oberfläche vorhanden sein kann. Somit können in der erfindungsgemäßen 3D-Gerüststruktur die Art, die Anzahl, der Ort, die räumliche Verteilung und der Funktionalisierungen sowie ihre Derivatisierungen und deren Beweglichkeit und Abstände von den Funktionalisierungen gezielt und nach Bedarf eingestellt werden.

Durch die Kombination von (i) bis (iv) sowie dem gezielten Einbau von Nanopartikel in gewünschte Gerüststrukturteile kann ausgehend von einer einzigen Spezies eines Vorläufermoleküls der Formel (I) bzw. (II) eine Vielzahl von unterschiedlichen Strukturen und Funktionalitäten sowie Kombinationen davon in ein und derselben erfindungsgemäßen 3D-Gerüststruktur erhalten werden.

Der Einfluss von topographischen und chemischen Substrateigenschaften auf das Zellmigrationsverhalten kann in mehreren Schritten untersucht werden. In einem Schritt kann die Topographie des Zellsubstrats von zweidimensionalen, flachen Schichten über quasidreidimensionale Säulenfelder bis hin zu dreidimensionalen kubischen Gitter-Strukturen variiert werden. In einem anderen Schritt kann die Wirkung der funktionalisierten Polymersysteme untersucht werden. In einem weiteren Schritt werden die Erkenntnisse der ersten Schritte kombiniert, und es erfolgt die Bildung dreidimensionaler Strukturen gemischter Funktionalisierung.

## Beispiele

I. Synthese von Ausführungsbeispiel 1

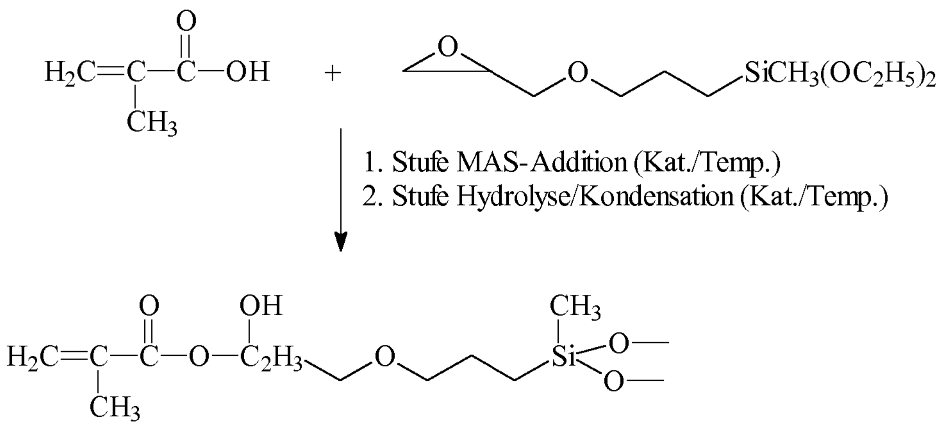


Die Synthese des Harzsystems mit $R_2$ = OH ist in DE 4416857 beschrieben. Zur Vorlage von 372,6 g 3-Glycidyloxypropylmethyldiethoxysilan werden unter trockener Atmosphäre Triphenylphosphin als Katalysator, BHT als Stabilisator und anschließend 142,1 g Methacrylsäure zugetropft und bei 85 °C gerührt. Nach Zugabe von Essigester als Lösungsmittel und $H_2O$ zur Hydrolyse mit HCl als Katalysator wird bei 30 °C gerührt. Die Aufarbeitung erfolgt nach ca. mehrtägigem Rühren durch mehrmaliges Ausschütteln mit wässrigem NaOH und mit Wasser und Filtration. Es wird zunächst abrotiert und anschließend mit Ölpumpenvakuum abgezogen. Es resultiert ein flüssiges Harz mit einer Viskosität von ca. 3 - 5 Pa·s bei 25 °C.

Das Harzsystem mit $R^2$ = -OH kann als allgemeine Grundstruktur für die weitere Funktionalisierung zu beispielsweise $R^2$ = $-CO_2H$, $-NR_2$ eingesetzt werden.

II. Synthese von Ausführungsbeispiel 2

(a) Die Synthese des Harzsystems erfolgt mit $R^2$ = $OH/CO_2H$ im Molverhältnis von 0,5:0,5. Die Lösung von 20,0 g (76 mmol) des Harzsystems aus Ausführungsbeispiel 1 und 3,8 g (38 mmol) Bernsteinsäureanhydrid in 15 ml THF

wird unter Rückfluss erhitzt. Eine Säuregruppe des Anhydrids geht mit der OH-Gruppe ein Esterbindung ein, die verbleibende Säuregruppe des Anhydrids stellt dann die gewünschte $CO_2H$-Gruppe dar. Nach vollständigem Umsatz erfolgt die Aufarbeitung (Abzug desTHF, Auflösen in Essigester, Ausschütteln mit $H_2O$, Abzug der flüchtigen Bestandteile). Es resultiert ein flüssiges Harz.

(b) Synthese des Harzsystems mit $R_2$ = OH/$CO_2H$ im Molverhältnis von 1,0 : 0,8 (Einsatzverhältnis; nach NMR-Auswertung im fertigen Produkt resultiert ein Verhältnis von 1,0 : 0,76). Die Lösung von 20,8 g (0,08 mol) des Harzsystems aus Ausführungsbeispiel 1 und 6,4 g (0,04 mol) Bernsteinsäureanhydrid in 10 ml THF und nach Zugabe von 1,8-Diazabicyclo[5.4.0]undec-7-en (= DBU) (0,01 mol DBU/mol Bernsteinsäureanhydrid) wird unter Rückfluss erhitzt. Nach vollständigem Umsatz erfolgt die Aufarbeitung (Abzug des THF, Auflösen in Essigester, Ausschütteln mit $H_2O$, Abzug der flüchtigen Bestandteile). Es resultiert ein flüssiges Harz.III. Synthese von Ausführungsbeispiel 3

Synthesestufe 1

Gemäß der Beschreibung in DE 103.49766.8

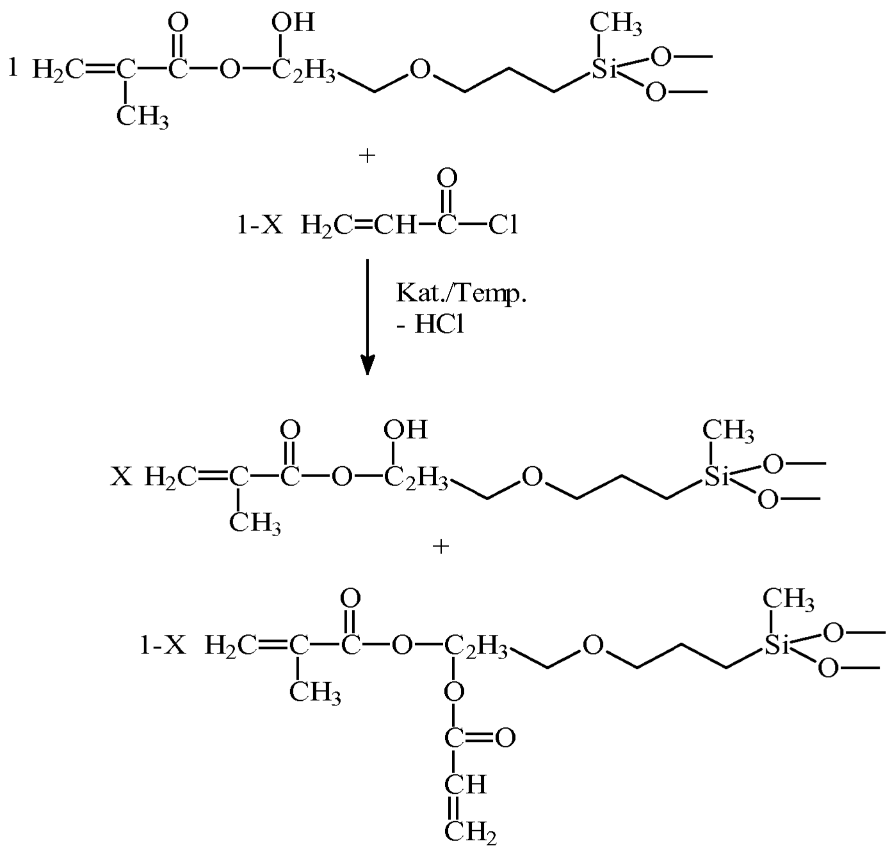


Grundharz A (X ≈ 0,61)

Zur Vorlage von 78,15 g (0,30 mol) des Harzsystems aus Ausführungsbeispiel 1 und 16,70 g Triethylamin in 300 ml THF als Lösungsmittel werden unter trockener Atmosphäre und Kühlung mittels Eisbades unter Rühren 11,72 g (0,130 mol) Acrylsäurechlorid zugetropft und bei Raumtemperature weitergerührt. Nach üblicher Aufarbeitung zur Abtrennung des bei der Addition entstehenden Aminhydrochlorids und säurehaltiger Nebenprodukte sowie Abziehen der flüchtigen Bestandteile mit Ölpumpenvakuum resultiert ein flüssiges Harz.

Synthesestufe 2

In der Abbildung ist nur der der acrylatmodifizierte Teil des Grundharzes A dargestellt.

Die Synthese des Harzsystems mit $R_2$ = OH/$NEt_2$ erfolgt im Molverhältnis von etwa 0,63:0,37. Zur Vorlage von

4,37 g von Grundharzsystem A werden unter Rühren die dem Acrylatanteil entsprechende Menge Diethylamin (0,5 g) zugesetzt und bei Raumtemperatur bis zur vollständigen Addition weiter gerührt. Die -CH=$CH_2$-Doppelbindungen des Acrylats werden teilweise mit Diethlamin zu -$CH_2$-$CH_2$-$NEt_2$ umgesetzt. Nach Abziehen der flüchtigen Bestandteile mit Ölpumpenvakuum resultiert ein flüssiges Harz.

Die vorstehenden Ausführungsbeispiele zeigen, dass die -OH-Gruppe der Verbindung aus Ausführungsbeispiel 1 zur weiteren Funktionalisierung mit -$CO_2$H-Gruppen und die (Meth)acrylgruppe zur anteiligen Funktionalisierung mit -$NR^3_2$-Gruppen ($R^3$ = -C2H5) eingesetzt werden kann.

Die Funktionalisierung mit -$CO_2$H-Gruppen kann gemäß Ausführungsbeispiel 2 so erfolgen, dass neben den -$CO_2$H-Gruppen auch noch -OH-Gruppen verbleiben, d. h. es resultiert ein System mit -$CO_2$H-und -OH-Gruppen, wobei unterschiedliche Anteile möglich sind.

Die anteilige Funktionalisierung mit -$NR^3_2$-Gruppen erfolgt gemäß Ausführungsbeispiel 3 so, das neben den -$NR^3_2$-Gruppen auch -OH-Gruppen vorliegen, d. h. es resultiert ein System mit -$NR^3_2$ -und -OH-Gruppen, wobei unterschiedliche Anteile möglich sind.

Somit sind flüssige Harzsysteme bzw. final gehärtete Systeme mit definierter makroskopischer Gerüststruktur in Verbindung mit unterschiedlichen Funktionalisierungsvarianten bei aber gleicher chemischer Grundstruktur möglich.

Anwendungsbeispiele

In den Anwendungsbeispielen wurden die in Tabelle 1 gezeigten Hybridpolymersysteme eingesetzt, die durch unterschiedliche chemische Gruppen (-OH bzw. -COOH/-OH bzw. - $N(C_2H_5)_2$/-OH) intrinsisch funktionalisiert wurden.

Tabelle 1: Funktionalisierungsgrade der im Anwendungsbeispiel verwendeten Hybridpolymere

| Funktionalisierung $R^2$ | Funktionalisierungsgrad |
|---|---|
| Hydroxy (-OH) | 100% |
| Carboxy (-COOH/-OH) | 50%/50% |
| Amin/Hydroxy (-$N(C_2H_5)_2$/-OH) | 39% / 61 % |

Anwendungsbeispiel 1: Farbstoffanbindung

Es wurde N-Hydroxysulfosuccinimid (Sulfo-NHS, Life Technologies GmbH, Darmstadt, Deutschland) verwendet, um den Farbstoff Alexa 647 (Alexa 647 Hydrazid, Life Technologies GmbH, Darmstadt, Deutschland) an die Oberfläche von Carboxy-funktionalisiertem Hybridpolymer anzubinden.

Dazu wurden 30 μm dicke Schichten des Carboxy-funktionalisierten Hybridpolymers auf ein Deckglas gerakelt. Als Referenz wurde eine identische Schicht aus einem rein Hydroxy-funktionalisierten Hybridpolymer hergestellt, bei dem keine Anbindung über das Kopplungsmolekül möglich ist.

Teile dieser Proben wurden mittels eines chemischen Kopplungsverfahrens behandelt, das selektiv an Carboxygruppen bindet, um den Farbstoff Alexa 647 anzubringen. Es wurde auf beiden Proben folgende Prozedur zur Farbstoffanbindung durchgeführt. Zur Aktivierung der Carboxygruppe wurden 0,4 mg EDC und 1,1 mg Sulfo-NHS in 1mL MES-Puffer (0,1M MES, 0,5M NaCl, pH 6,0) gelöst, 200 mL dieser Lösung auf die Probe pipettiert und für 30 min bei Raumtemperatur zur Reaktion gebracht. Anschließend wurde die Probe mehrmals mit PBS-Lösung gespült und die Alexa647-Farbstofflösung (0,1mg in 500 μL PBS) aufpipettiert. Nach zwei Stunden wurde die Reaktion mit Hydroxylamine-HCL (Life Technologies GmbH, Darmstadt, Deutschland) unterdrückt, die Probe mit PBS gespült und über Nacht darin eingelegt, um nicht reagierte Substanzen zu lösen. Zum Vergleich wurden Teile dieser Proben nicht mit diesem Verfahren behandelt.

An beiden Proben wurden konfokale 3D-Fluoreszenzaufnahmen aufgenommen (Leica TCS, SP8X, Leica Microsystems GmbH, Wetzlar, Deutschland). Dabei wurde jeweils der Übergang des Polymers auf das unbeschichtete Deckglas untersucht, um den Effekt der Anbindung mittels Fluoreszenz zu verdeutlichen. Jede Probe wurde sowohl an der behandelten Stelle als auch an einer unbehandelten Stelle untersucht. Nur auf der behandelten Stelle der Carboxy-funktionalisierten Probe konnte eine starke Fluoreszenz von Alexa 647 nachgewiesen werden. An deren unbehandelten Stellen sowie bei beiden Messungen an der Hydroxy-funktionalisierten Probe konnte nur die sehr schwache Autofluoreszenz des Polymers sowie Grenzflächenstreuung detektiert werden.

Diese Untersuchung zeigt zum einen, dass die Carboxygruppen der intrinsischen Funktionalisierung chemisch an der Oberfläche zugänglich sind und zum anderen, dass sie dazu genutzt werden können, um ortsselektiv Farbstoffe, Proteine oder andere bioaktiven Stoffe auf einfachem Wege anzubinden.

Anwendungsbeispiel 2: Zellmigrationsmessungen

Es wurden Zellmigrationsmessungen auf Schichten von zwei Hybridpolymersystemen untersucht, die durch unterschiedliche chemische Gruppen (-OH bzw. -N($C_2H_5$)$_2$/-OH) intrinsisch funktionalisiert wurden (siehe Tabelle 1). Diese wurden mit Zellmigrationsmessungen auf Borosilikatglasdeckgläsern verglichen, die eine weit verbreitete Referenzoberfläche darstellt. Dieselben Messungen wurden in Säulenstrukturen aus den zwei Polymersystemen durchgeführt, sowie in Säulenstrukturen in denen einige Säulen aus dem Polymer -OH und andere aus dem Polymer -N($C_2H_5$)$_2$/-OH bestehen.

Dem Präpolymer wurden 2 Gew.-% UV-Initiator (Irgacure® 369, Ciba Geigy, 2-Benzyl-2-dimethylamino-1-(4-morpholinophenyl)-butanon-1) zugegeben, um ein photovernetzbares bzw. photostrukturierbares Material zu erhalten. Als Substrat für die Schichten wurden Präzisionsmikroskopdeckgläser (Borosilikatglas, No. 1.5H, A. Hartenstein, Deutschland) verwendet, deren Oberfläche mittels 3-(Trimethoxysilyl)propylmethacrylat (Sigma-Aldrich Chemie GmbH, Deutschland) silanisiert worden war, um die Haftung zu erhöhen. Darauf wurde das unvernetzte Präpolymer aufgetropft und mit einem weiteren Deckglas abgedeckt und bei einer Temperatur von 50°C mit einem Gewicht von 4 kg für 20 min zusammengepresst. Anschließend wurde das Präpolymer zwischen den Deckgläsern durch UV-Einstrahlung ausgehärtet (10 min, BK 850, Beltron®) und das nichtsilanisierte Deckglas entfernt (siehe Figur 1).

Die quasi-3D- und die 3D-Gerüststrukturen wurden mittels Zwei-Photonen Polymerisation hergestellt. Dabei wird ein gepulster Femtosekundenlaser (Chameleon Ultra II, Coherent Inc., USA) mit einer Wellenlänge von 705 nm mit einem Luftobjektiv (60x, CFI Plan Apo Lambda 0.95, Nikon, Japan) in das Harz hineinfokussiert und es kommt im Bereich des Objektivfokus zu einer Vernetzungsreaktion. Durch dreidimensionale Bewegung lassen sich die gewünschten Strukturen erzeugen. Anschließend wird der unvernetzte Anteil des Präpolymers durch einen Entwicklungsschritt in 50 % Isopropanol und 50 % 4-Methyl-2-pentanon (10 min, Sigma-Aldrich Chemie GmbH, Deutschland) gelöst, wobei nur die vernetzte 3D-Gerüststruktur erhalten bleibt. Um den Grad der Polymervernetzung zu erhöhen, wurden die Strukturen mittels UV-Bestrahlung endgehärtet (5 min, BK 850, Beltron®) (siehe Figur 2).

Um die Proben herzustellen, in denen beide Polymersysteme vereint sind, wird der beschriebene Prozessschritt zweimal hintereinander für jedes Polymer durchgeführt.

Alle Zellmigrationsmessungen wurden mit transfizierten D. *discoideum* Zellen (Dr. Günther Gerisch, Max Planck Institut für Biochemie, München, Deutschland) durchgeführt, die das grün fluoreszierende Protein (GFP) exprimieren, das homogen im Zytoplasma verteilt vorliegt (FreeGFP).

Die Fluoreszenzaufnahmen wurden an einem inversen Mikroskop (Eclipse Ti, Nikon, Japan), mit einem 20x Luftobjektiv (CFI Plan Apo 20x, Nikon, Japan) und einer EMCCD Kamera (iXon3 897, Andor Technology Ltd., U.K) bei einer Auflösung von 512x512 Pixeln aufgenommen. Für die Zeitreihenaufnahmen wurden alle 8 Sekunden für 45 min Bilder aufgenommen.

Die Aufnahmen wurden mit der Software Fiji bearbeitet und hierbei das Autofluoreszenzsignal der Polymerstrukturen durch eine Referenzaufnahme ohne Zellen entfernt und ein binäres Bild erzeugt. Daraus wurden mit Hilfe der Software Cell Evaluator die Massenschwerpunkte der Zellen ermittelt und so die Zelltrajektorien über der Zeit bestimmt. Diese Daten wurden mittels einem eigens entwickelten Matlab Algorithmus einer lokalen Mean-Squared-Displacement-Analyse (MSD-Analyse) (Arcitzet D et al. (2012) Soft matter 8:1473-1481; Gorelashvili M, Emmert M, Hodeck KF, Heinrich D (2014) New J. Phys. 16:75012; Emmert M, Witzel P, Rothenburger-Glaubitt M, Heinrich D (2017) RSC Adv. 7:5708-5714) unterzogen, um das Migrationsverhalten in gerichtete und ungerichtete Phasen zu unterteilen sowie die Geschwindigkeit und weitere Parameter zu ermitteln (vgl. Figur 3). Dazu wird in einem Intervall T um jeden Zeitpunkt t das lokale Mean-Squared-Displacement (MSD) ($\Delta R2(\tau)$) in Abhängigkeit von der Verzögerungszeit $\tau$ berechnet. Durch nichtlineare Ausgleichsrechnung mit einer Potenzfunktion wird der MSD-Exponent $\alpha$ bestimmt:

$$\langle \Delta R^2(\tau)\rangle = \langle \left(R(t_i+\tau) - R(t_i)\right)^2 \rangle_{t_i - \frac{T}{2} < t_i < t_i + \frac{T}{2}} = A\,\tau^{\alpha}$$

Anhand des $\alpha$-Exponenten lassen sich Zelltrajektorien in die beiden typischen Bewegungsmodi amöboider Zellmigration unterteilen (Figur 3, rechts). Für $\alpha \lesssim 1{,}75$ wird die Bewegung zum Zeitpunkt $t_i$ als ungerichtet klassifiziert, für $\alpha \gtrsim 1{,}75$ wird der Trajektorienabschnitt dem gerichteten Bewegungsmodus zugeordnet.

Anwendungsbeispiel 3: Zellmigrationsmessungen in quasi-3D-Strukturen mit lokal unterschiedlicher Funktionalisierung

Nachdem der Einfluss auf das Zellmigrationsverhalten durch die drei unterschiedlich intrinsisch funktionalisierten Hybridpolymere und der Unterschied bzgl. 2D und quasi 3D gezeigt werden konnte, wurden erneut Proben mit unterschiedlichen Funktionalitäten (-OH und -N($C_2H_5$)$_2$/-OH) hergestellt. Die Proben wurden in einem Schachbrettmuster angeordnet (Figur 8A), um die Berührungsfläche und -häufigkeit der Zellen mit den Strukturoberflächen zu analysieren.

In Figuren 8B und 8C sind diese Häufigkeiten in Abhängigkeit der Berührungsfläche aufgetragen. Für die Hydroxy-funktionalisierten Strukturen ist sowohl die Gesamtanzahl von über 12626 ca. 49% höher als bei den Amin-funktionalisierten Strukturen. Außerdem ist der Verlauf des Histogramms deutlich zu größeren Berührungsflächen hin verschoben. Die Zellen kontaktieren demnach deutlich häufiger die Hydroxystrukturen und dies zudem mit einer größeren Kontaktfläche. Dieses Ergebnis unterstreicht die Möglichkeit, durch die intrinsische Funktionalisierung gezielt chemisch heterogene Proben herzustellen und dadurch Einfluss auf das Zellverhalten zu nehmen.

Einfluss des E-Moduls und der Oberflächenrauheit auf das Zellverhalten

Um strukturelle Randeffekte auszuschließen, wurden für die E-Modul Messungen Säulen mit einem Durchmesser von 50 μm strukturiert und deren Oberfläche mit einem Microindenter (Fischerscope® HM2000, Helmut Fischer GmbH) vermessen. Die Indentierungstiefe von 1,5 μm wurde nach 20 s erreicht, gefolgt von 5 s Relaxationszeit und der vollständigen Retraktion innerhalb weiterer 20s. Die Ergebnisse in Tabelle 2 zeigen, dass die resultierenden mechanischen Eigenschaften nicht von der intrinsischen Funktionalisierung abhängen und daher nicht zu den Unterschieden im Zellverhalten beitragen können.

Tabelle 2: Verschiedene Härteparameter von 2PP strukturierten Säulen unterschiedlicher Funktionalisierung, ermittelt mit einem Mikroindenter.

| Funktionalisierung | E-Modul (GPa) | Vickers Härte |
|---|---|---|
| OH | 2,31 ± 0,12 | 13,1 ± 0,5 |
| COOH | 2,28 ± 0,04 | 13,4 ± 0,7 |
| Amin | 2,34 ± 0,25 | 12,6 ± 1,5 |

Die Oberflächenrauheit von Amin-funktionalisierten Säulen wurde mittels Rasterkraftmikroskopie bestimmt. Dazu wurden diese nach der Strukturierung umgelegt, damit die seitliche Oberfläche, mit denen die Zellen bei den Migrationsversuchen in Kontakt stehen, vermessen werden konnte (Figur 9A). Eine typische Rasterkraftmessung einer Säule ist in Figur 9B gezeigt, aus der die Oberflächenrauheit nach Abzug der Säulenkrümmung hervorgeht (Figur 9C). Bei fünf vermessenen Säulen ergibt sich ein Mittelwert von $R_A$ = (6,8 ± 1,6) nm. Die Rasterelektronenmikroskopaufnahmen in Figur 9D zeigen, dass kein Unterschied zwischen den drei untersuchten Materialien besteht und somit auch kein Einfluss auf das Zellverhalten genommen wird. Es wurde also gezeigt, dass nur die Oberflächenfunktionalisierung zum veränderten Zellverhalten führt.

Direkter Nachweis der funktionellen Gruppen mittels Röntgenphotoelektronenspektroskopie:

Röntgenphotoelektronenspektroskopie (XPS) beruht auf der Herauslösung von Photoelektronen mittels Röntgenstrahlung aus dem zu untersuchenden Festkörper und kann dazu genutzt werden, um die Elementzusammensetzung der Probe zu bestimmen. Da die mittlere freie Weglänge von Elektronen im Festkörper sehr gering ist, beträgt die Informationstiefe wenige Nanometer und erlaubt Aussagen über die chemische Zusammensetzung der Oberfläche.

XPS wurde genutzt, um die chemischen Gruppen der intrinsischen Funktionalisierung an der Oberfläche nachzuweisen. Hierzu wurde eine Scheibe mit einem Durchmesser von 5 mm mittels 2PP strukturiert und mit einer S-Probe von Surface Science Instruments untersucht. Die Auswertung erfolgte mit der Software Hawk Data Analysis 7, v7.03.04. In Figur 10 sind für alle drei untersuchten Polymere jeweils die Kohlenstoff- und Stickstoffbereiche der 1s Orbitale des Spektrums dargestellt. Bei den Carboxy-funktionalisierten Proben (Figur 10B) sind Bindungsenergien um 289 eV deutlich ausgeprägter als bei den Hydroxy-funktionalisierten (Figur 10A), die Carboxygruppen zugeordnet werden können. An Stickstoff gebundene Kohlenstoffatome treten zwischen 286 - 286,5 eV auf und führen bei der Amin-funktionalisierten Probe zu einer entsprechenden Erhöhung im Spektrum (Figur 10 C).

Bindungsenergien des 1s-Orbitals von Stickstoff konnte nur für die Amin-funktionalisierte Probe nachgewiesen werden (Figur 10F).

Da es sich bei XPS um eine sehr oberflächensensitive Untersuchungsmethode handelt, kann davon ausgegangen werden, dass sowohl die Carboxy- als auch die Amingruppen an den TPA Strukturen zugänglich sind.

**Patentansprüche**

1. Dreidimensionale Gerüststruktur, die Gerüststrukturteile aus einem Polymersystem und zwischen den Gerüststrukturteilen fluidgefüllte offene Räume mit einem Mindestmaß von 50 nm in jeder Raumrichtung aufweist, wobei das Polymersystem durch Behandlung eines Ausgangsmaterials mit elektromagnetischer Strahlung herstellbar ist, wobei das Ausgangsmaterial Vorläufermoleküle der Formel (I) oder anorganische Polymere davon enthält:

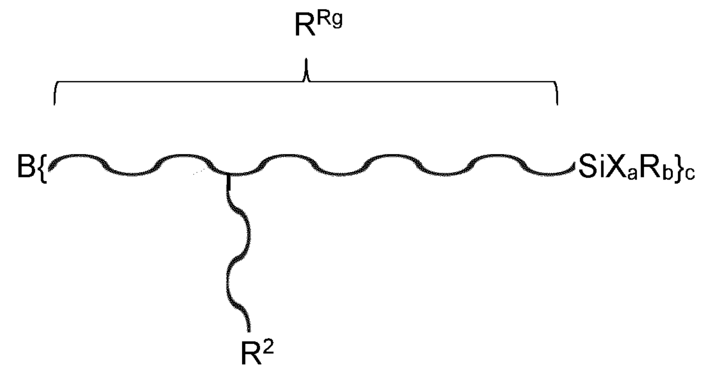

Formel (I)

worin die Reste und Indices folgende Bedeutung haben:

B ist ein geradkettiger oder verzweigter oder cyclischer organischer Rest mit mindestens einer C=C-Doppelbindung und 4 bis 100 Kohlenstoffatomen;
X ist Wasserstoff, Halogen, Hydroxy, Alkoxy, Acyloxy, Alkylcarbonyl, Alkoxycarbonyl oder $NR^4_2$;
R ist Alkyl, Alkenyl, Aryl, Alkylaryl oder Arylalkyl;
$R^2$ ist OH, COOH, $NR^4_2$, $NR^4_2H^+$ oder SH oder ein Derivat davon, wobei durch die Derivatisierung $OR^5$, $COOR^5$, $NR^4R^5$, $NR^4R^5H^+$ oder $SR^5$ entsteht, wobei $R^5$ der Rest eines Biofunktionsmoleküls oder Farbstoffs ist;
$R^4$ ist Wasserstoff, Alkyl, Aryl oder Alkylaryl;
$R^{Rg}$ ist das Rückgrat eines an das Si-Atom gebundenen geradkettigen oder verzweigten Kohlenwasserstoffes, wobei das Rückgrat durch Heteroatome oder heteroatomhaltige Gruppen unterbrochen sein kann;
a = 1, 2 oder 3;
b = 0, 1 oder 2;
a+b = 3;
c = 1, 2, 3 oder 4.

**2.** Dreidimensionale Gerüststruktur nach Anspruch 1, wobei die Vorläufermoleküle der Formel (I) eine Struktur der Formel (II) aufweisen:

$$B\{A\text{-}(Z)_d\text{-}R^1\text{-}R^3\text{-}SiX_aR_b\}_c$$
$$|$$
$$(O\text{-}CO\text{-}R\text{-})_eR^2$$

Formel (II)

worin die Reste und Indices folgende Bedeutung haben:

B, X, R, $R^2$, $R^4$, a, b, a+b und c haben die gleiche Bedeutung wie in Formel (I);
$R^1$ und $R^3$ sind unabhängig voneinander Alkylen, Arylen oder Alkylenarylen mit jeweils 1 bis 20 Kohlenstoffatomen, wobei diese Reste durch O, S, NR oder NH unterbrochen sein können,
A ist O, S oder NH für d = 1 und Z = CO; oder A ist O, S, NH oder COO für d = 1;
Z ist $CHR^4$; oder A ist O, S, NH oder COO für d = 0; und
e = 0 oder 1.

**3.** Dreidimensionale Gerüststruktur nach Anspruch 1 oder 2, wobei das Polymersystem auf mindestens eine erste Art von Vorläufermolekülen der Formel (I) oder anorganischen Polymeren davon sowie auf mindestens eine zweite Art von Vorläufermolekülen oder anorganischen Polymeren davon zurückgeht, wobei sich die zweite Art von der ersten Art mindestens oder ausschließlich darin unterscheidet, dass $R^2$ Wasserstoff ist; oder wobei das Polymersystem auf mindestens zwei Arten von Vorläufermolekülen oder anorganischen Polymeren davon zurückgeht, die sich mindestens oder ausschließlich in der Gruppe $R^2$ unterscheiden.

**4.** Dreidimensionale Gerüststruktur nach Anspruch 3, wobei der Unterschied in der Gruppe $R^2$ darin besteht, dass in

der einen Art des Vorläufermoleküls $R^2$ OH, COOH, $NR^4_2$, $NR^4_2H^+$ oder SH ist und in der anderen Art des Vorläufermoleküls $R^2$ ein Derivat von $R^2$ der ersten Art ist.

**5.** Dreidimensionale Gerüststruktur nach einem der vorstehenden Ansprüche, wobei das Derivat von $R^2$ auf ein Biofunktionsmolekül oder auf einen Abstandshalter mit angebundenem Biofunktionsmolekül zurückgeht.

**6.** Dreidimensionale Gerüststruktur nach einem der vorstehenden Ansprüche, wobei das Polymersystem Nanopartikel enthält.

**7.** Dreidimensionale Gerüststruktur, die zwei Gerüststrukturteile enthält, die in einem der vorstehenden Ansprüche beschriebene Polymersysteme enthalten, wobei sich die Polymersysteme in mindestens einem Merkmal unterscheiden, das aus der Gruppe ausgewählt ist, die aus der Art des Vorläufermoleküls der Formel (I) oder eines anorganischen Polymers davon; dem Anteil des Vorläufermoleküls der Formel (I) oder eines anorganischen Polymers davon an den gesamten photostrukturierbaren Verbindungen; dem Anteil eines Vorläufermoleküls, das mit Ausnahme von $R^2$=H dem eingesetzten Vorläufermolekül der Formel (I) entspricht; dem Gehalt an Nanopartikeln; dem Anteil des Vorläufermoleküls der Formel (I), worin $R^2$ ein Derivat ist; und der Art des Derivats an $R^2$ besteht.

**8.** Dreidimensionale Gerüststruktur nach einem der vorstehenden Ansprüche, worin die Polymersysteme aus einer einzigen Art eines Vorläufermoleküls herstellbar sind, mit der Ausnahme, dass sich die Vorläufermoleküle in dem Vorhandensein oder der Art der Derivatisierung an $R^2$ oder in beiden unterscheiden können.

**9.** Dreidimensionale Gerüststruktur nach einem der Ansprüche 1 bis 7, die Gerüststrukturteile aus einem Polymersystem, das im Ausgangsmaterial erste Vorläufermoleküle der Formel (I) oder anorganische Polymere davon enthält, und Gerüststrukturteile aus einem Polymersystem umfasst, das im Ausgangsmaterial von den ersten verschiedene zweite Vorläufermoleküle der Formel (I) oder anorganische Polymere davon enthält.

**10.** Dreidimensionale Gerüststruktur nach einem der vorstehenden Ansprüche, die mindestens eine erste Struktureinheit einer aus dem Bereich von 10 $\mu$m bis 100 mm ausgewählten ersten Dicke und von der ersten Struktureinheit abzweigende zweite Struktureinheiten einer jeweils aus dem Bereich von 100 nm bis 1000 $\mu$m ausgewählten zweiten Dicke enthält, wobei an den Abzweigungen die zweite Dicke höchstens die Hälfte der ersten Dicke ist.

**11.** Dreidimensionale Gerüststruktur nach einem der vorstehenden Ansprüche für die Verwendung zur Wechselwirkung mit biologischen Zellen in vitro oder für eine medizinische Verwendung in vivo.

**12.** Verfahren zum Herstellen einer dreidimensionalen Gerüststruktur nach einem der Ansprüche 1 bis 11, welches die folgenden Schritte umfasst:

(a) Bereitstellen eines in Anspruch 1 oder Anspruch 2 beschriebenen photostrukturierbaren Ausgangsmaterials,
(b) Photostrukturierung des in Schritt (a) bereitgestellten Ausgangsmaterials unter Bildung einer dreidimensionalen Gerüststruktur und
(c) Entfernen des nicht umgesetzten Ausgangsmaterials.

**13.** Verfahren nach Anspruch 12, welches einen ersten Durchgang der Schritte (a) bis (c) mit einer ersten Position der Photostrukturierung und einen zweiten Durchgang der Schritte (a) bis (c) mit einer von der ersten Position verschiedenen zweiten Position der Photostrukturierung unter Bildung von zwei Teilstrukturen der dreidimensionalen Gerüststruktur umfasst.

**14.** Verfahren nach Anspruch 13, wobei sich die Ausgangsmaterialien des ersten Durchgangs von denen des zweiten Durchgangs in mindestens einem Merkmal unterscheiden, das aus der Gruppe ausgewählt ist, die aus der Art des Vorläufermoleküls der Formel (I) oder eines anorganischen Polymers davon; dem Anteil des Vorläufermoleküls der Formel (I) oder eines anorganischen Polymers davon an den gesamten photostrukturierbaren Verbindungen; dem Anteil eines Vorläufermoleküls, das mit Ausnahme von $R^2$=H dem eingesetzten Vorläufermolekül der Formel (I) entspricht; dem Gehalt an Nanopartikeln; dem Anteil des Vorläufermoleküls der Formel (I), worin $R^2$ ein Derivat ist; und der Art des Derivats an $R^2$ besteht.

**15.** Verfahren nach Anspruch 13 oder 14, wobei nur eine einzige Art des Vorläufermoleküls der Formel (I) oder eines anorganischen Polymers davon eingesetzt wird, mit der Ausnahme, dass sich die Vorläufermoleküle in dem Vorhandensein oder der Art der Derivatisierung an $R^2$ oder in beiden unterscheiden können, und wobei diese Art in

beiden Durchgängen gleich oder verschieden ist.

## Claims

1. A three-dimensional scaffold structure comprising scaffold structure parts made of a polymer system and fluid-filled open spaces between the scaffold structure parts with a minimum extent of 50 nm in each spatial direction, the polymer system being obtainable by treating a starting material with electromagnetic radiation, the starting material containing precursor molecules of the formula (I) or inorganic polymers thereof:

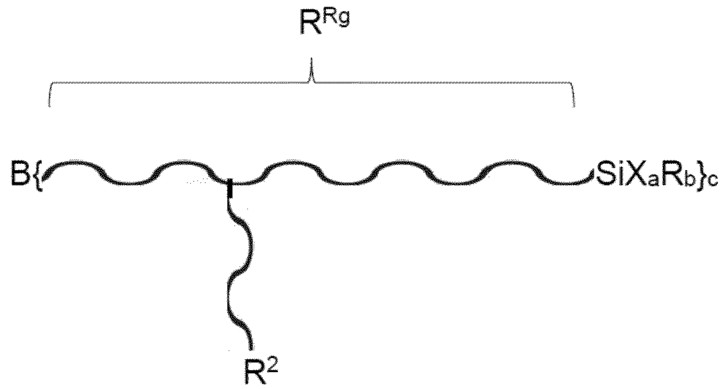

Formula (I)

wherein the radicals and indices have the following meaning:

B is a straight-chain or branched or cyclic organic radical having at least one C=C double bond and 4 to 100 carbon atoms;
X is hydrogen, halogen, hydroxy, alkoxy, acyloxy, alkylcarbonyl, alkoxycarbonyl or $NR^4_2$;
R is alkyl, alkenyl, aryl, alkylaryl or arylalkyl;
$R^2$ is OH, COOH, $NR^4_2$, $NR^4_2H^+$ or SH or a derivative thereof, wherein the derivatization results in $OR^5$, $COOR^5$, $NR^4R^5$, $NR^4R^5H^+$ or $SR^5$, wherein $R^5$ is the residue of a biofunctional molecule or dye;
$R^4$ is hydrogen, alkyl, aryl or alkylaryl;
$R^{Rg}$ is the backbone of a straight-chain or branched hydrocarbon bonded to the Si atom, where the backbone may be interrupted by heteroatoms or heteroatom-containing groups;
a = 1, 2 or 3;
b = 0, 1 or 2;
a+b = 3;
c = 1, 2, 3 or 4.

2. The three-dimensional scaffold structure according to claim 1, wherein the precursor molecules of formula (I) have a structure of formula (II):

$$B\{A\text{-}(Z)_d\text{-}R^1\text{-}R^3\text{-}SiX_aR_b\}_c$$ Formula (II)

$$|$$

$$(O\text{-}CO\text{-}R\text{-})_eR^2$$

in which the radicals and indices have the following meaning:

B, X, R, $R^2$, $R^4$, a, b, a+b and c have the same meaning as in formula (I);
$R^1$ and $R^3$ are independently of one another alkylene, arylene or alkylenearylene each having 1 to 20 carbon atoms, it being possible for these radicals to be interrupted by O, S, NR or NH,
A is O, S or NH for d = 1 and Z = CO; or A is O, S, NH or COO for d = 1;
Z is $CHR^4$; or A is O, S, NH or COO for d = 0; and
e = 0 or 1.

3. The three-dimensional scaffold structure according to claim 1 or 2, wherein the polymer system is based on at least one first type of precursor molecules of formula (I) or inorganic polymers thereof and at least one second type of precursor molecules or inorganic polymers thereof, wherein the second type differs from the first type at least or exclusively in that $R^2$ is hydrogen; or wherein the polymer system is based on at least two types of precursor molecules or inorganic polymers thereof which differ at least or exclusively in the group $R^2$.

4. The three-dimensional scaffold structure according to claim 3, wherein the difference is that $R^2$ is OH, COOH, $NR^4{}_2$, $NR^4{}_2H^+$ or SH in one type of precursor molecule and $R^2$ is a derivative of $R^2$ of the first type in the other type of precursor molecule.

5. The three-dimensional scaffold structure according to any of the preceding claims, wherein the derivative of $R^2$ is derived from a biofunctional molecule or from a spacer having a bound biofunctional molecule.

6. The three-dimensional scaffold structure according to any of the preceding claims, wherein the polymer system contains nanoparticles.

7. The three-dimensional scaffold structure comprising two scaffold structure parts comprising polymer systems described in any of the preceding claims, said polymer systems differing in at least one characteristic selected from the group consisting of the type of precursor molecule of formula (I) or an inorganic polymer thereof; the proportion of the precursor molecule of formula (I) or an inorganic polymer thereof in the total photostructurable compounds; the proportion of a precursor molecule corresponding, with the exception of $R^2$=H, to the precursor molecule used of formula (I); the nanoparticle content; the proportion of the precursor molecule of formula (I) in which $R^2$ is a derivative; and the nature of the derivative of $R^2$.

8. The three-dimensional scaffold structure according to any of the preceding claims, wherein the polymer systems are producible from a single type of precursor molecule, except that the precursor molecules may differ in the presence or type of derivatization at $R^2$ or in both.

9. The three-dimensional scaffold structure according to any of claims 1 to 7, comprising scaffold structure parts of a polymer system containing in the starting material first precursor molecules of formula (I) or inorganic polymers thereof and scaffold structure parts of a polymer system containing in the starting material different second precursor molecules of formula (I) or inorganic polymers thereof, different from the first precursor molecules.

10. The three-dimensional scaffold structure according to any of the preceding claims, comprising at least a first structural unit of a first thickness selected from the range from 10 $\mu$m to 100 mm and second structural units of a second thickness selected from the range from 100 nm to 1000 $\mu$m each branching off from the first structural unit, the second thickness at the branches being at most half the first thickness.

11. The three-dimensional scaffold structure according to any of the preceding claims for use for interaction with biological cells in vitro or for medical use in vivo.

12. A method for fabricating a three-dimensional scaffold structure according to any one of claims 1 to 11, comprising the steps of:

(a) providing a photostructurable source material described in claim 1,
(b) photostructuring the starting material provided in step (a) to form the three-dimensional scaffold structure; and
(c) removing the unreacted starting material.

13. The method according to claim 12 which comprises a first pass of steps (a) to (c) having a first position of photostructuring and a second pass of steps (a) to (c) having a second position of photostructuring different from the first position to form two partial structures of the three-dimensional scaffold structure.

14. The method according to claim 13, wherein the starting materials of the first pass differ from those of the second pass in at least one characteristic selected from the group consisting of the type of precursor molecule of formula (I) or an inorganic polymer thereof; the proportion of the precursor molecule of formula (I) or an inorganic polymer thereof in the total photostructurable compounds; the proportion of a precursor molecule corresponding, with the exception of $R^2$=H, to the precursor molecule used of formula (I); the nanoparticle content; the proportion of the precursor molecule of formula (I) in which $R^2$ is a derivative; and the nature of the derivative of $R^2$.

**15.** The method according to claim 13 or 14, wherein only a single type of precursor molecule of formula (I) or an inorganic polymer thereof is used except that the precursor molecules may differ in the presence or type of derivatization at $R^2$ or in both, and wherein said type is the same or different in both passes.

## Revendications

**1.** Structure d'échafaudage tridimensionnel, qui présente des parties de structure d'échafaudage composées d'un système de polymère et d'espaces ouverts remplis de fluide entre les parties de structure d'échafaudage avec une dimension minimale de 50 nm dans chaque direction spatiale, dans laquelle le système de polymère peut être produit par un traitement d'un matériau de départ avec un rayonnement électromagnétique, dans laquelle le matériau de départ contient des molécules précurseurs de formule (I) ou des polymères inorganiques de celles-ci :

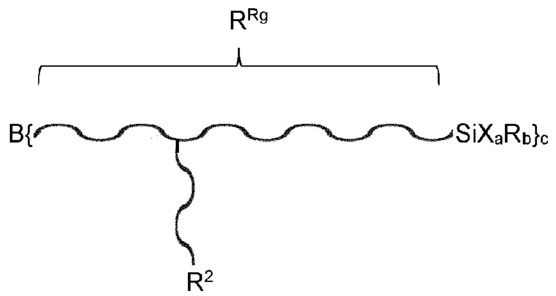

Formule (I)

où les résidus et les indices ont la signification suivante :

B est un résidu organique linéaire ou ramifié ou cyclique avec au moins une liaison double C=C et 4 à 100 atomes de carbone ;
X est de l'hydrogène, halogène, hydroxy, alkoxy, acyloxy, alkylcarbonyle, alkoxycarbonyle ou $NR^4_2$ ;
R est de l'alkyle, alcényle, aryle, akylaryle ou arylalkyle ;
$R^2$ est OH, COOH, $NR^4_2$, $NR^4_2H^+$ ou SH ou un dérivé de ceux-ci, dans laquelle $OR^5$, $COOR^5$, $NR^4R^5$, $NR^4R^5H^+$ ou $SR^5$ se forment par dérivatisation, dans laquelle $R^5$ est le résidu d'une molécule biofonctionnelle ou d'un colorant ;
$R^4$ est de l'hydrogène, alkyle, aryle ou alkylaryle ;
$R^{Rg}$ est la colonne vertébrale d'un hydrocarbure linéaire ou ramifié lié à l'atome Si, dans laquelle la colonne vertébrale peut être interrompue par des hétéroatomes ou des groupes contenant des hétéroatomes ;
a = 1, 2 ou 3 ;
b = 0, 1 ou 2 ;
a + b = 3 ;
c = 1, 2, 3 ou 4.

**2.** Structure d'échafaudage tridimensionnel selon la revendication 1, dans laquelle les molécules précurseurs de formule (I) présentent une structure de formule (II) :

$$\begin{array}{l} B\{A\text{-}(Z)_d\text{-}R^1\text{-}R^3\text{-}SiX_aR_b\}_c \\ \qquad\qquad\quad | \\ \qquad\qquad (O\text{-}CO\text{-}R\text{-})_eR^2 \end{array}$$

Formule (II)

où les résidus et les indices ont la signification suivante :

B, X, R, $R^2$, $R^4$, a, b, a+b et c ont la même signification qu'en formule (I) ;
$R^1$ et $R^3$ sont des alkyles, aryles ou alkyles aryles indépendants les uns des autres avec respectivement 1 à 20 atomes de carbone, dans laquelle ces résidus peuvent être interrompus par O, S, NR ou NH,
A est O, S ou NH pour d = 1 et Z = CO ; ou A est O, S, NH ou COO pour d = 1 ;
Z est $CHR^4$ ; ou A est O, S, NH ou COO pour d = 0 ; et
e = 0 ou 1.

3. Structure d'échafaudage tridimensionnel selon la revendication 1 ou 2, dans laquelle le système de polymère dérive d'au moins une première forme de molécules précurseurs de formule (I) ou de polymères inorganiques de celles-ci et d'au moins une seconde forme de molécules précurseurs ou de polymères inorganiques de celles-ci, dans laquelle la seconde forme se distingue au moins ou exclusivement de la première forme par le fait que $R^2$ est de l'hydrogène ; ou dans laquelle le système de polymère dérive d'au moins deux formes de molécules précurseurs ou de polymères inorganiques de celles-ci qui se différentient au moins ou exclusivement dans le groupe $R^2$.

4. Structure d'échafaudage tridimensionnel selon la revendication 3, dans laquelle la différence dans le groupe $R^2$ consiste en ce que dans l'une forme de la molécule précurseur $R^2$ est OH, COOH, $NR^4_2$, $NR^4_2H^+$ ou SH et dans l'autre forme de la molécule précurseur $R^2$ est un dérivé de $R^2$ de la première forme.

5. Structure d'échafaudage tridimensionnel selon une des revendications précédentes, dans laquelle le dérivé de $R^2$ dérive d'une molécule biofonctionnelle ou d'une séquence d'espacement avec une molécule biofonctionnelle liée.

6. Structure d'échafaudage tridimensionnel selon une des revendications précédentes, dans laquelle le système de polymère contient des nanoparticules.

7. Structure d'échafaudage tridimensionnel, qui contient deux parties de structure d'échafaudage qui contiennent des systèmes de polymère décrits dans une des revendications précédentes, dans laquelle les systèmes de polymère se différencient par au moins une caractéristique qui est choisie dans le groupe qui est constitué de : la forme de la molécule précurseur de formule (I) ou d'un polymère inorganique de celle-ci ; la part de la molécule précurseur de formule (I) ou d'un polymère inorganique de celle-ci au niveau de toutes les liaisons photostructurables ; la part d'une molécule précurseur qui correspond à la molécule précurseur de formule (I) utilisée, à l'exception de $R^2$=H ; la teneur en nanoparticules ; la part de la molécule précurseur de formule (I), où $R^2$ est un dérivé ; et de la forme du dérivé en $R^2$.

8. Structure d'échafaudage tridimensionnel selon une des revendications précédentes, où les systèmes de polymère peuvent être produits à partir d'une seule forme de molécule précurseur, à l'exception que les molécules précurseurs peuvent se différencier en présence ou dans la forme de la dérivatisation en $R^2$ ou dans les deux.

9. Structure d'échafaudage tridimensionnel selon une des revendications 1 à 7, qui comprend des parties de structure d'échafaudage composées d'un système de polymère, qui contient dans le matériau de départ des premières molécules précurseurs de formule (I) ou des polymères inorganiques de celles-ci, et des parties de structure d'échafaudage composées d'un système de polymère qui contient dans le matériau de départ des secondes molécules précurseurs de formule (I) différentes des premières ou des polymères inorganiques de celles-ci.

10. Structure d'échafaudage tridimensionnel selon une des revendications précédentes, qui contient au moins une première unité de structure d'une première épaisseur choisie dans la plage de 10 $\mu$m à 100 mm et des secondes unités de structure dérivées de la première unité de structure et d'une seconde épaisseur respectivement choisie dans la plage de 100 nm à 1 000 $\mu$m, dans laquelle au niveau des dérivations la seconde épaisseur représente au plus la moitié de la première épaisseur.

11. Structure d'échafaudage tridimensionnel selon une des revendications précédentes pour une utilisation en interaction avec des cellules biologiques in vitro ou pour une utilisation médicale in vivo.

12. Procédé destiné à produire une structure d'échafaudage tridimensionnel selon une des revendications 1 à 11, lequel comprend les étapes suivantes :

(a) préparation d'un matériau de départ photostructurable décrit dans la revendication 1 ou la revendication 2,
(b) photostructuration du matériau de départ préparé à l'étape (a) en formant une structure d'échafaudage tridimensionnel et
(c) élimination du matériau de départ non transformé.

13. Procédé selon la revendication 12, lequel comprend un premier passage des étapes (a) à (c) avec une première position de la photostructuration et un second passage des étapes (a) à (c) avec une seconde position de photostructuration différente de la première position en formant deux structures partielles de la structure d'échafaudage tridimensionnel.

**14.** Procédé selon la revendication 13, dans lequel les matériaux de départ du premier passage se différencient de ceux du second passage par au moins une caractéristique qui est choisie dans le groupe qui est constitué de : la forme de la molécule précurseur de formule (I) ou d'un polymère inorganique de celle-ci ; la part de la molécule précurseur de formule (I) ou d'un polymère inorganique de celle-ci au niveau de toutes les liaisons photostructurables ; la part d'une molécule précurseur qui correspond à la molécule précurseur de formule (I) utilisée à l'exception de $R^2$=H ; la teneur en nanoparticules ; la part de la molécule précurseur de formule (I), où $R^2$ est un dérivé ; et la forme du dérivé en $R^2$.

**15.** Procédé selon la revendication 13 ou 14, dans lequel une seule forme de molécule précurseur de formule (I) ou d'un polymère inorganique de celle-ci est utilisée, à l'exception que les molécules précurseurs peuvent se différencier en présence ou dans la forme de la dérivatisation en $R^2$ ou dans les deux et dans lequel cette forme est identique ou différente dans les deux passages.

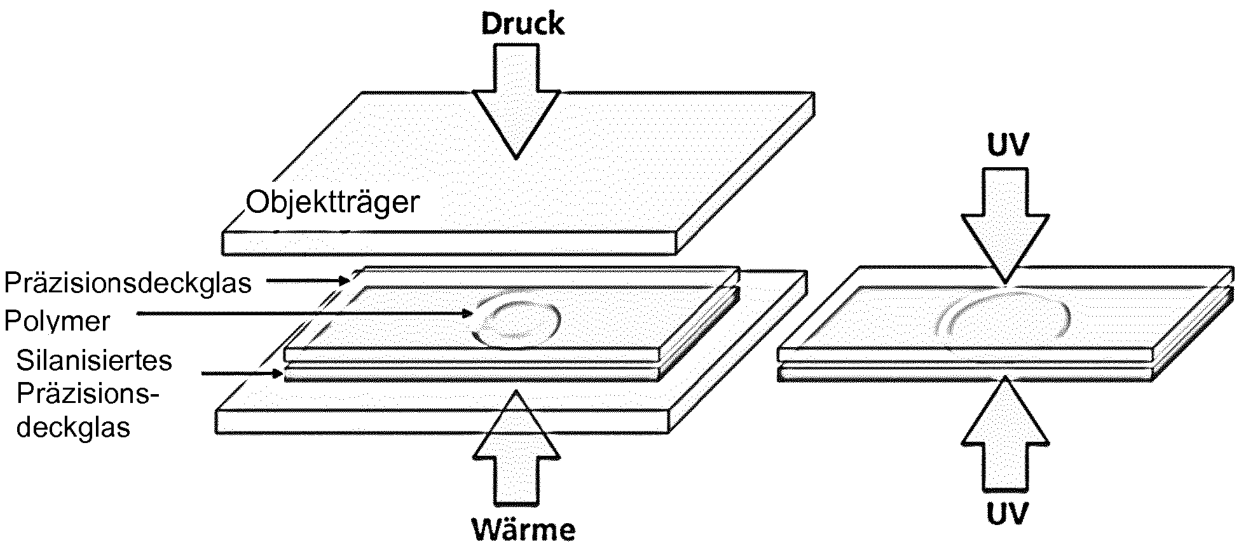


Figur 1

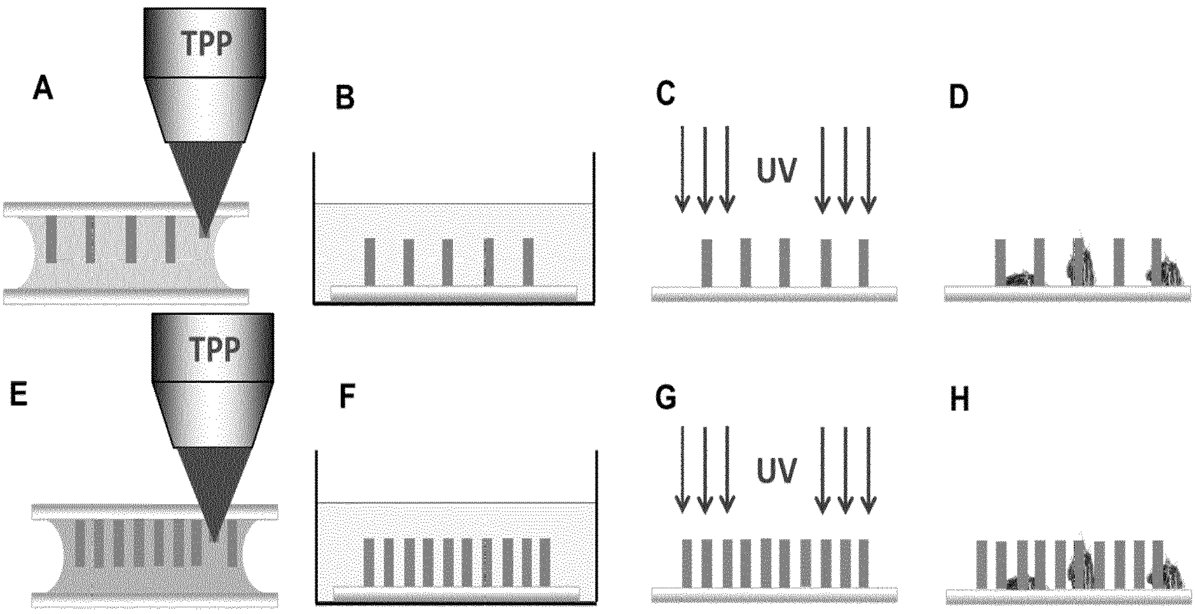


Figur 2

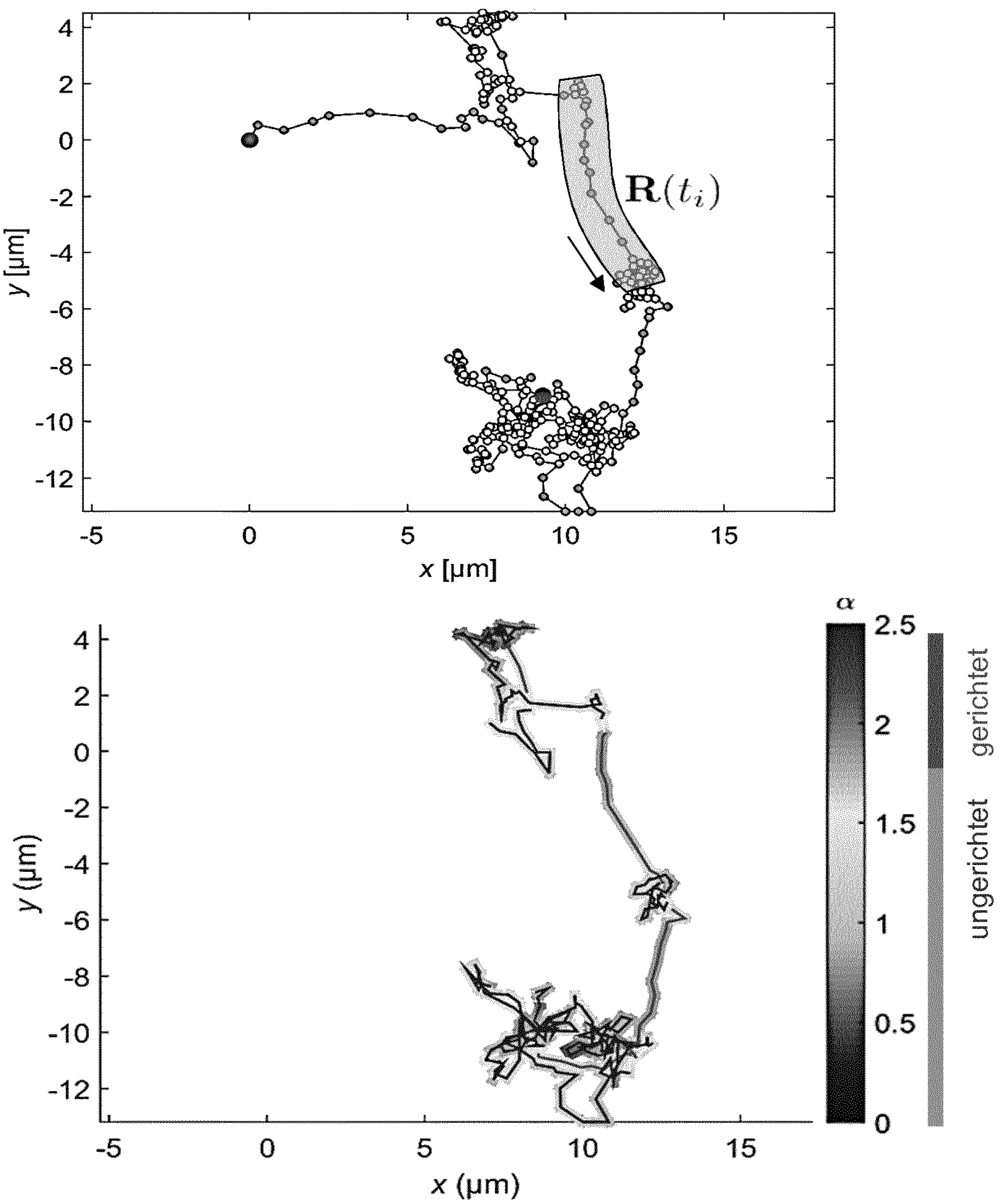


Figur 3

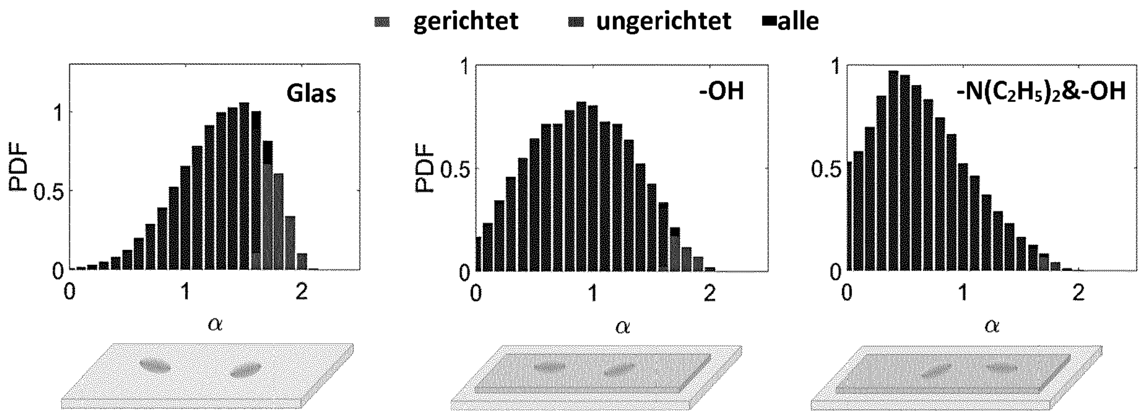


Figur 4

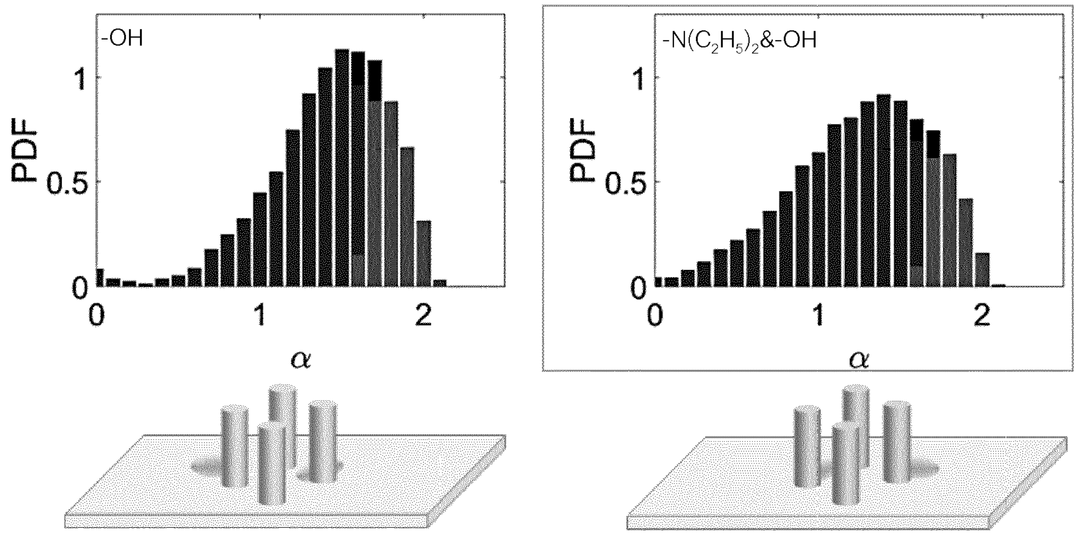


Figur 5

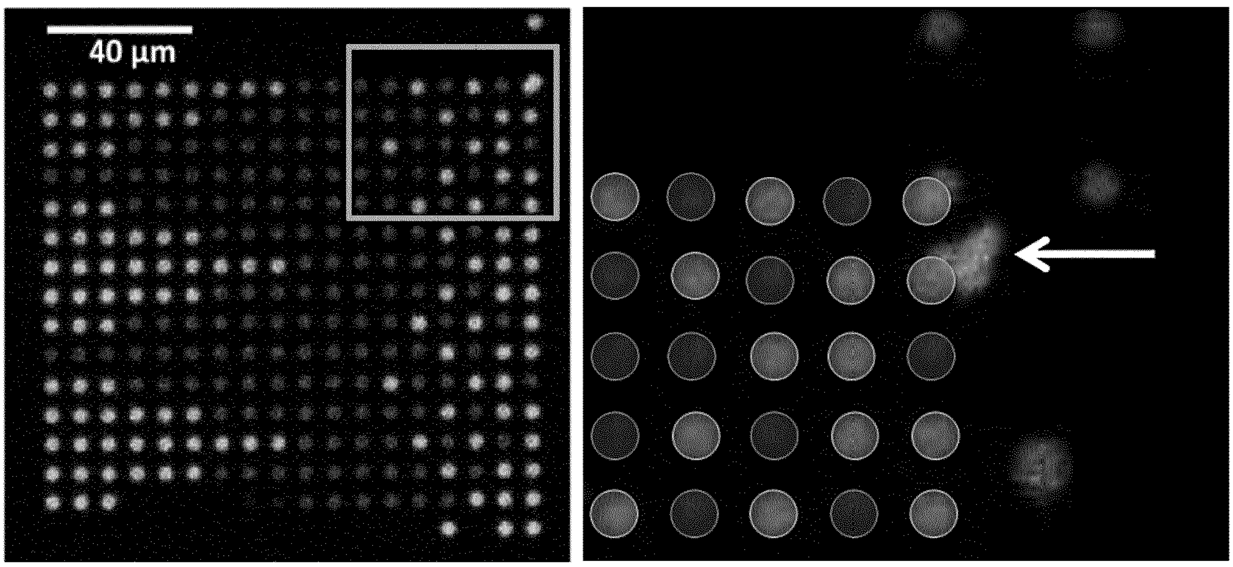


Figur 6

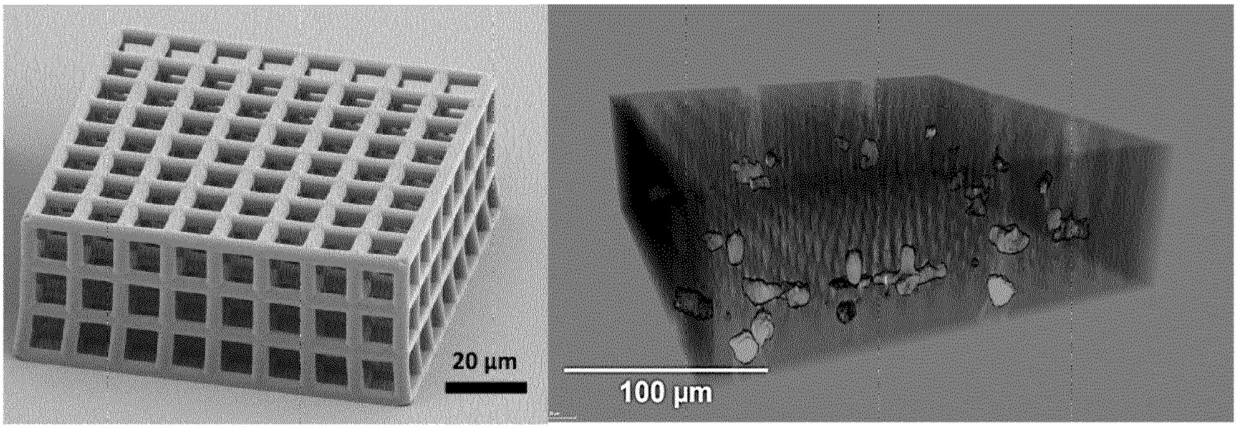


Figur 7

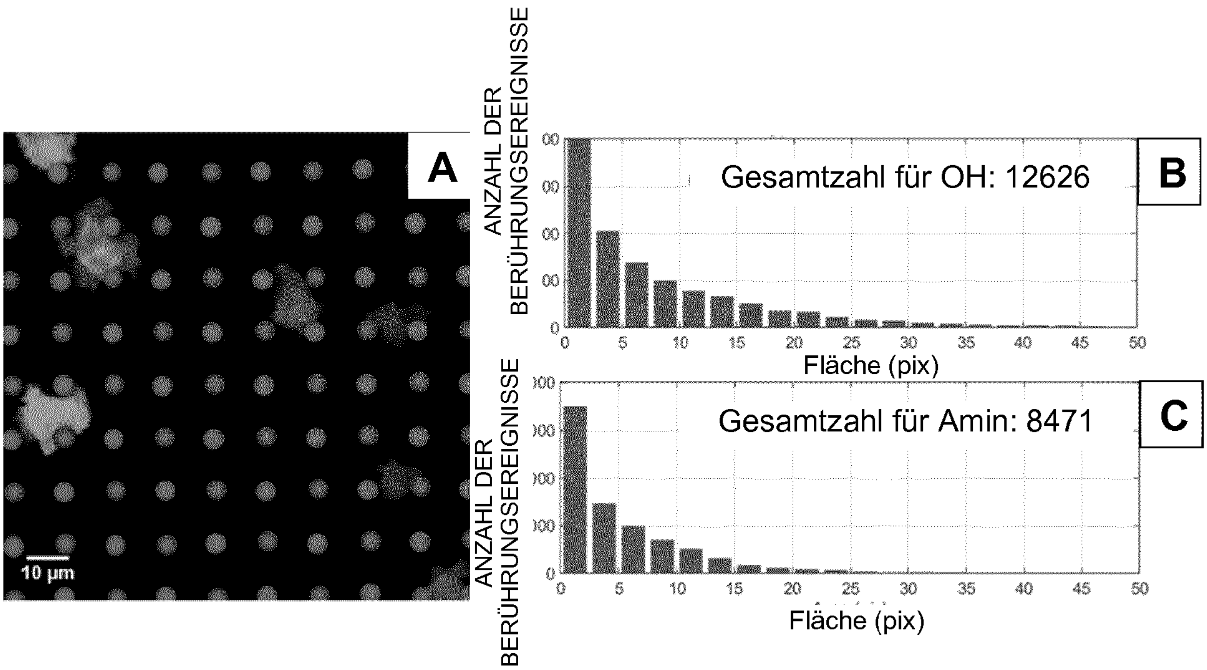


Figur 8

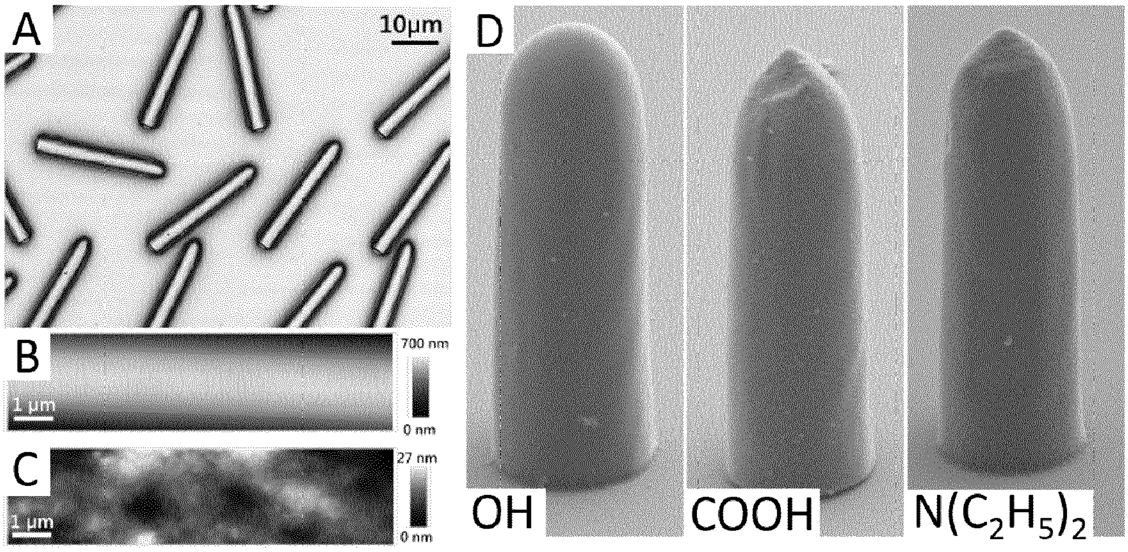


Figur 9

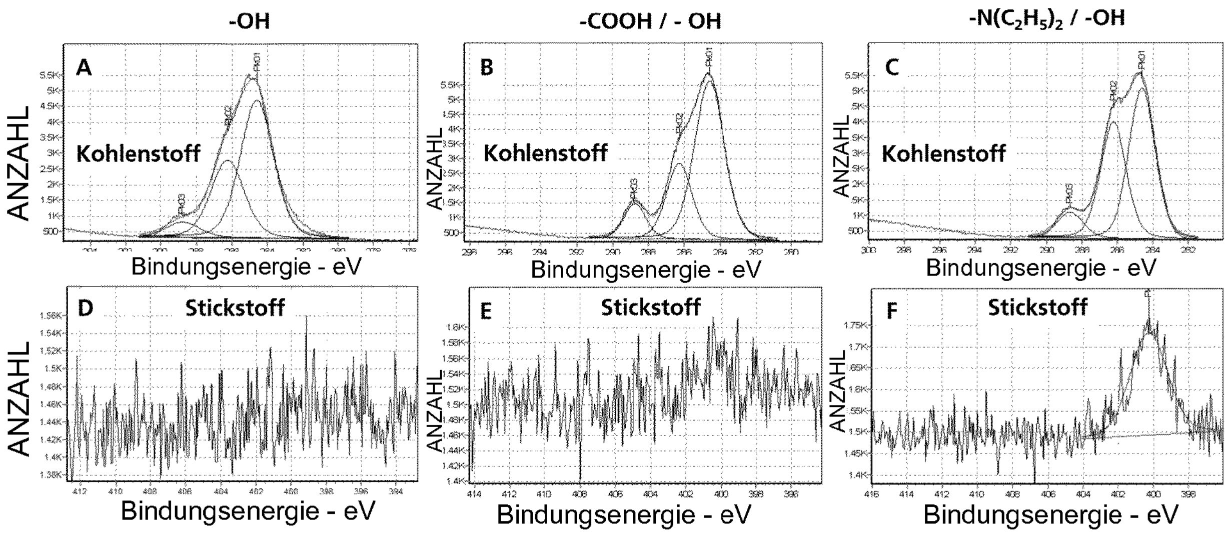


Figur 10

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102017101823 A1
- EP 2870209 B1
- EP 0682033 A2
- DE 4416857


### In der Beschreibung aufgeführte Nicht-Patentliteratur


- **SCHLIE S et al.** Three-dimensional cell growth on structures fabricated from ORMOCER by two-photon polymerization technique. *Journal of biomaterials applications,* 2007, vol. 22, 275-287
- **REKSTYTE S.** Direct Laser Fabrication of Composite Material 3D Microstructured Scaffolds. *JLMN,* 2014, vol. 9, 25-30
- **KLEIN F et al.** Two-component polymer scaffolds for controlled three-dimensional cell culture. *Advanced materials (Deerfield Beach, Fla.),* 2011, vol. 23, 1341-1345
- **RICHTER B et al.** Guiding Cell Attachment in 3D Microscaffolds Selectively Functionalized with Two Distinct Adhesion Proteins. *Advanced materials,* 2017, 29
- **ARCITZET D et al.** *Soft matter,* 2012, vol. 8, 1473-1481
- **GORELASHVILI M ; EMMERT M ; HODECK KF ; HEINRICH D.** *New J. Phys.,* 2014, vol. 16, 75012
- **EMMERT M ; WITZEL P ; ROTHENBURGER-GLAUBITT M ; HEINRICH D.** *RSC Adv.,* 2017, vol. 7, 5708-5714